# EUROPEAN PATENT SPECIFICATION

(11) **EP 1 680 509 B1**
(45) Date of publication and mention of the grant of the patent: **04.12.2019**
(21) Application number: 04793835.2
(22) Date of filing: 22.10.2004
(51) Int. Cl.: C12P 7/04, C12P 7/06, C12P 7/16, C12P 7/20, C12P 7/24, C12P 7/54

(54) **METHOD OF PRODUCING HYDROCARBONS AND OXYGEN-CONTAINING COMPOUNDS FROM BIOMASS INCLUDING FERMENTATION WITH ADDITION OF AMINO ACIDS (LEU, ILE, VAL)**
VERFAHREN ZUR HERSTELLUNG VON KOHLENWASSERSTOFFEN UND SAUERSTOFFHALTIGEN VERBINDUNGEN AUS BIOMASSE UMFASSEND FERMENTATION UNTER ZUSATZ VON AMINOSÄUREN (LEU, ILE, VAL)
PROCÉDÉ DE PRODUCTION D'HYDROCARBURES ET DE COMPOSÉS CONTENANT DE L'OXYGÈNE À PARTIR DE BIOMASSE COMPRENANT UNE FERMENTATION AVEC ADDITION D'ACIDES AMINÉS (LEU, ILE, VAL)

(30) Priority: 24.10.2003 SE 0302800; 24.10.2003 US 513583 P
(43) Date of publication of application: 19.07.2006
(73) Proprietor: Swedish Biofuels AB, 102 27 Stockholm (SE)
(72) Inventor: GOLUBKOV, Igor, S-181 31 Lidingö (SE)
(74) Representative: Brann AB
(86) International application number: PCT/SE2004/001534
(87) International publication number: WO 2005/040392

(56) References cited:
- US-A- 4 326 032
- US-A- 5 173 429
- US-A1- 2002 160 469
- US-B1- 6 509 180
- DATABASE CAPLUS [Online] SHIN ENERGY SOGO KAIHATSU KIKO: 'Manufacture of ethanol with zymomonas cultured in media containing specific amino acids', XP002984131 Retrieved from STN Database accession no. 1988:189891 & JP 63 017 696 A
- DATABASE EPODOC [Online] EUROPEAN PATENT OFFICE, THE HAGUE, NL; 25 January 1988 (1988-01-25), YAGI ET AL: "Fermentation of alcohol", Database accession no. JP63017696A
- DATABASE WPI [Online] THOMSON SCIENTIFIC, LONDON, GB; 25 January 1988 (1988-01-25), YAGI ET AL: "Alcoholic fermentation process", Database accession no. 1988-061236

## Description

### Technical field of the invention

The present invention generally relates to biochemical and chemical industry, and more particularly to a method which can be used in fermenting carbohydrate substrates of plant origin for producing C₁-C₅ alcohols, and for synthesis of higher alcohols, other oxygen-containing compounds and hydrocarbons as well as for the production of motor fuel components from biomass. Since C₆ and higher alcohols, ethers, acetals, and higher hydrocarbons are not obtainable by a direct biochemical route, it is proposed to synthesize these using known chemical reactions, wherein by-products of fermentation are used as raw materials for said synthesis. The invention is defined in the claims and is characterized in that the amino acids leucine, isoleucine, or valine, or a mixture thereof, is/ are added to the aqueous carbohydrate substrate in an amount providing a content of amino nitrogen of from 120 to 420 mg/l of fermentation medium.

### Background art

Obtaining alcohols and other oxygen-containing compounds by fermentation of carbohydrates is known since long ago [Brief Chemical Encyclopaedia, Moscow, 1967] and used industrially mainly for producing ethanol. However, even the most advanced process for biochemical production of ethanol allows for conversion of only about half of the source carbohydrate substrate into the final commercial alcohol. The remaining part of carbohydrates is used for maintaining the vital functions of microorganisms and is converted into carbon dioxide. When it comes to other alcohols or other oxygen-containing compounds, such as ketones or acids [H.G. Schlegel. Allgemeine Mikrobiologie, 1985], the known biochemical processes allow conversion of raw materials into the final products to an even lesser extent. Considerable part of the carbohydrate substrate in these processes is converted into by-products. The obtaining of hydrocarbons by means of biochemical methods is also well known since long ago [H.G. Schlegel. Allgemeine Mikrobiologie, 1985]. However, biogas obtained in fermentation of the waste of livestock farming or in decomposition of biomass by bacteria contains mainly methane. Production of hydrocarbons and oxygen-containing compounds from synthesis gas, which in turn originates from biomass, also seems problematic. At present there is no industrial process for producing hydrocarbons and oxygen-containing compounds using synthesis gas obtained from biomass. Synthesis gas obtained from coal, petroleum and natural gas is used industrially for producing oxygen-containing compounds [Reaction of hydroformylation, Kirk-Othmer Encyclopaedia, 3rd edition, v. 19, N.Y., 1982]. These processes are widely used by the industry for producing aldehydes, alcohols and many other oxygen-containing compounds originating from said materials. Methods for producing hydrocarbons from synthesis gas are also well known and used industrially [Fisher-Tropsch reaction, Kirk-Othmer Encyclopaedia, 3rd edition, v. 19, N.Y., 1982]. However nothing is known about using synthesis gas obtained from biological raw materials in these processes.

There are various methods for intensifying the production of ethyl alcohol, such as by introducing new types of microorganisms, characterized by a higher speed of fermentation and wider range of utilization of the carbohydrates substrates, by using continuous processes of fermentation or processes of cell immobilizing, or by effective processing of new and traditional types of raw material, providing widening of the range of the raw materials and deeper assimilation of the raw-material components. The productivity of the fermentation process in these methods can reach up to 10-15 litres of ethanol per cubic meter of the fermentor volume per hour, and specific speed of fermentation can reach up to 2.5-3.0 litres of ethanol per 1 kilogram of the yeast biomass on a dry weight basis per hour, with a yield of ethanol from the fermented carbohydrates in terms of weight of up to 49-50% (the theoretic value is 51%).

The prior art discloses a method for preparation of the grain starch-containing raw material for alcohol fermentation (RU 2145354, C12P7/06, 1998). The method includes cleaning of the grain from admixtures, mixing with water, thermal treatment, adding of enzymes, acid, and saccharification. After cleaning the grain is divided into the floury kernel and husk. Further processing of the raw material is carried out in two streams: the floury kernel is mixed with water to reach 19-21% mass part of humidity and treated thermally by extrusion. Then, after mixing with water, amylolytic enzymes and acid are added in an amount providing optimal pH value for the particular enzyme used. This is followed by saccharification, after which the husk is mixed with water to 21-23% of the mass water content and at least 2% by mass of alkali is added. The material is then treated thermally with addition of the acid in an amount providing the optimum pH value for the specific enzyme used. Then the cellulolytic enzymes are added and saccharification is carried out. Thereafter, both streams are brought together and directed to the fermentation.

There is a known method for producing ethyl alcohol from grain raw material (RU 2127760, C12P7/06, 1997). The method specifies the following steps: the grain is cleaned of husk, crushed, mixed with the liquid fraction, treated thermally, then the amylolytic enzymes carrying out the enzymatic hydrolysis of starch are added, the mass is sterilized, cooled down, enzymatic complex is added, followed by saccharification and cooling down to the fermentation temperature. The obtained wort-mash is distilled to obtain ethyl alcohol and distillery dreg. The total amount of the distillery dreg obtained is divided into two streams, one of which is further separated into two streams, one of which is re-directed to the stage of thermal processing of the grain separated from husk, where it is used as a liquid phase in the mixture with water; the other stream, after 15-16 hours after the start of fermentation, is directed to each of the fermentors fermenting the mass at the fermentation stage in separate streams in the amount of 15-20% of the volume of the fermenting medium. The remaining stream of the distillery dreg is taken out of the process in the mixture with the separated husk for using as a fodder product.

Disadvantages of the above-described methods are their low specific speed of fermentation (1.5-2.0 1/kg*h) and low C₃-C₅ alcohols yield. C₃-C₅ alcohols (fusel oil) are byproduct of ethanol production from the plant raw material. The yield of C₃-C₅ alcohols in the production of ethanol by known methods is 0.2-0.6% of ethanol. In the production of a food-grade ethanol C₃-C₅ alcohols are an unwanted admixture and should be thoroughly removed by rectification and purification. All technologic means in the process of a food-grade ethanol production, starting from the raw material preparation and finishing with the rectification, aim at minimizing the formation of fusel oil or at its maximum removal.

Collecting and storing fusel oil for the subsequent qualified processing and using is non-expedient due to its low yield. Modern methods for utilization of fusel oil propose either its incineration in a burner in admixture with fuel oil (Klimovski D.I, Smirnov V.N. "Alcohol Technology, Moscow, 1967) or using fusel oil as a raw material for producing isoamyl alcohol by distillation in a rectification unit (Russian patent RU 2109724, C07C 31/125, 1996). Recently, the methods for producing fuel-grade ethanol from carbohydrates of plant origin have gained a great importance. There are different known methods for using the products of fermentation of carbohydrate substrates of plant origin: ethyl alcohol and C₃-C₅ alcohols as a motor fuel or components of motor fuels for internal combustion engines. In this case ethyl alcohol is mainly used as a fuel component, while C₃-C₅ alcohols are used as an octane-boosting additive for the fuel, or as a component in chemical synthesis for obtaining diesel fuel (Russian Patent 2155793, C10L1/18, 2000 "High octane additive for obtaining automotive gasoline", Russian Patent RU 2106391, C10L 1/18, 1995 "Composition of hydrocarbon fuel").

US 2002/0160469 A1 discloses a method for production of ethanol wherein either a pasteurized, hydrolyzed soy product, or a pasteurized, hydrolyzed autolyzed yeast product is contacted with ethanologenic bacteria, especially Erwinia, Klebsiella, Xan-thomonas or Escherichia. In Example 2 yeast autolysate has been used in an amount corresponding to a FAN content of about 8.4 mg/l in the fermentation medium.

JP 63017696 discloses a method of avoiding the decline in fermentation speed which results at high concentrations of glucose, i.e. 12-30 % or more when using Zymomonas bacteria, e.g. for alchohol production. According to JP 63017696, when specific amino acids are added to culture mediums with a high glucose concentration and high salt content, in which obstruction to fermentation usually occurs, such obstruction could be removed, and significant improvements in fermentation performance (quantity and yield of alcohol) using Zymomonas bacteria thereby became possible. Especially glutamic acid, serine or aspartic acid are suitable for use. The amount of amino acid should be not less than 0.05 % (w/v) of the medium, and preferably 0.1-0.5 % (w/v).

In the light of the above, ethyl alcohol production with an increased yield of C3-C5 alcohols would offer a possibility to widen the range of various types of motor fuels produced by processing "green" carbohydrate raw material. The total yield of fusel oil obtained in fermentation depends on the quality of carbohydrate substrate and the method of fermentation and is generally 0.2-0.6% of absolute ethyl
alcohol.

### Summary of the invention

We have developed a new method for obtaining hydrocarbons and oxygen-containing compounds from biomass or products originating from biomass. The process can be carried out in several steps and may include also biosynthesis of methane, carbon dioxide, acetaldehyde, acetone, lower C₁-C₅ alcohols, and glycerine for producing unsaturated hydrocarbons from said alcohols, obtaining of synthesis gas, including using methane and carbon dioxide, interaction of unsaturated hydrocarbons with synthesis gas, condensation of the obtained aldehydes, hydrogenation of the obtained unsaturated aldehydes into alcohols, and converting saturated alcohols into saturated hydrocarbons. Besides that, the aldehydes can be used for obtaining acids, which are then converted into
esters.

The aldehydes can also be used for the synthesis of acetals. The alcohols can also be converted into ethers. Moreover, C₁-C₅ alcohols and glycerine obtained in biosynthesis can first be converted into aldehydes, which are then condensed into higher unsaturated aldehydes, which in turn are hydrogenated into higher saturated alcohols.

The present invention, as defined in the claims, refers to a method of producing hydrocarbons and oxygen-containing compounds from biomass, comprising the steps of: preparation of an aqueous carbohydrate substrate with a carbohydrate concentration of 3-20% comprising a source of nitrogen, fermenting the substrate using yeast
or a bacterium selected from Clostridium acetobutylicum and Clostridium butylicum to an overall concentration of 1.5-10% of the following products, C₁-C₅ alcohols, glycerine (= glycerol), acetaldehyde, acetic acid and acetone, and separation of desired products from the fermentation medium, characterized in that, as a source of nitrogen, the amino acids leucine, isoleucine, or valine, or a mixture thereof is added to the aqueous carbohydrate substrate in an amount providing a content of amino nitrogen of from 120 to 420 mg/l of fermentation medium. The method finds use in the biochemical and chemical industry and can be used for fermenting carbohydrate substrates of plant origin for producing C₁-C₅ alcohols, and for synthesis of higher alcohols, other oxygen-containing compounds and hydrocarbons as well as for the production of motor fuel components from biomass. Since C₆ and higher alcohols, ethers, acetals, and higher hydrocarbons are not obtainable by a direct biochemical route, it is proposed to synthesize these using known chemical reactions, wherein the source raw materials for said synthesis are:
- Synthesis gas produced from carbon dioxide obtained in biomass fermentation and from methane obtained in fermentation of the amino acids-containing distillery dreg after extraction of alcohol, and/or from various products and wastes obtained in biomass processing, including processing of wood, production of grain, or production of vegetable oils;
- C₁-C₅ alcohols produced by the present method using amino acids as a bio-catalyst at the fermentation stage. The amino acids are leucine, isoleucine, valine, or a mixture of these amino acids extracted from the yeast autolysate after separation of asparagine and ammonium;
- Glycerine (glycerol) produced by the present method and/or by saponification of fats. It is proposed to use the glycerine for producing higher hydrocarbons and oxygen-containing compounds to increase the extent of utilization of the renewable raw material, including for the purpose of motor fuel production;
- Acetaldehyde and acetone produced by the present method.

It is proposed to use carbon dioxide or a mixture of carbon dioxide and oxygen in the processes of alcohol oxidation into aldehydes and aldehydes into fatty acids. At the stage of aldehydes condensation and to increase the yield of the higher hydrocarbons we propose to use besides the aldehydes obtained from alcohols the furfural obtained by hydrolysis of the pentosane-containing raw material. At the stage of etherification and to increase the yield of higher esters we propose to use along with the fatty acids, obtained from aldehydes, C₂-C₆ fatty acids produced by biosynthesis, as well as the acids obtained in the saponification of fats and extracted from the tall oil. To increase the yield of higher esters we also propose to use terpenes at the etherification stage.

To increase the extent of the biomass conversion in the synthesis of hydrocarbons and oxygen-containing compounds we propose to produce methanol using carbon dioxide obtained in the enzymatic processing of biomass or a mixture of carbon dioxide and hydrogen. Methanol obtained from carbon dioxide is then directed to the production of higher hydrocarbons and oxygen-containing compounds.

To increase the extent of the biomass conversion in the synthesis of hydrocarbons and oxygen-containing compounds it is proposed to use carbon dioxide obtained in the enzymatic processing of biomass. Besides carbon dioxide the said carbon oxide production can use wastes of the grain production, wood processing, turf, and lignin obtained in hydrolysis of the cellulose-containing raw material.

Production of synthesis gas can use as a raw material wastes of the production of grain, vegetable oils, wastes of wood processing, including pulp and wood coal, as well as by-products and wastes obtained in C₁-C₅ alcohols biosynthesis, biosynthesis of glycerine, acetaldehyde, acetone, C₂-C₆ acids, and by-products obtained in the chemical processing of the aforesaid oxygen-containing compounds. The following can be used for producing synthesis gas: gaseous and liquid products obtained in biomass pyrolysis, furfural, turpentine, colophony, tall oil, fusel oil, vegetable oils, and wastes obtained in the processing of said products.

It is also proposed to use carbon oxide obtained by the present method from the stage of fermentation, or from various types of biomass, and hydrogen, obtained from water by known methods, for the production of synthesis gas.

It is proposed to use synthesis gas produced by the present method for obtaining hydrocarbons and oxygen-containing compounds by the Fisher-Tropsch method and by methods based on hydroformylation.

Of course, the present method for producing hydrocarbons and oxygen-containing compounds from biomass or products originating from biomass allows using some source compounds of non-biologic origin. For example, in the production of synthesis gas along with carbon dioxide obtained in biosynthesis can be used hydrogen originating from petroleum, natural gas or coal. However the greatest effect is achieved when the source compounds are substances originating from renewable raw material. This is a possibility to obtain products needed for vital activities of humans from the raw materials currently not used to a full extent, but continuously reproduced by nature in contrast to petroleum, gas and coal, the reserves of which decrease continuously.

The present invention, as defined in the claims, aims at solving the following problems:
- To increase the yield of C₃-C₅ alcohols;
- To increase the specific speed of carbohydrate substrates fermentation;
- To utilize the protein-containing waste of the alcohol production;
- To produce higher oxygen-containing hydrocarbons and non oxygen-containing hydrocarbons, including those having C₄ and more carbon atoms in the molecule, from biomass and using the raw material obtained by biochemical methods;
- To utilize in the said production carbon dioxide obtained in biosynthesis of lower alcohols, acids and hydrocarbons; glycerine obtained in saponification of fats; furfural obtained in hydrolysis of the pentosane-containing raw material; fatty acids obtained in biosynthesis, fat saponification and extracted from tall oil, resin and gases obtained in pyrolysis of wood;
- To increase the rate of direct utilization of biomass for synthesis of higher alcohols, other oxygen-containing compounds, and higher hydrocarbons for motor fuel production from biomass.

### Detailed description of the invention

The present method, as defined in the claims, for fermentation of carbohydrate substrates allows to increase the yield of C₃-C₅ alcohols to a level of 0.65-3.1% of ethyl alcohol with a simultaneous increase of the specific speed of fermentation of carbohydrate substrates to 4.0 1/kg*h. This is accomplished as follows.

While carrying out the alcohol fermentation it is necessary to add to the carbohydrate substrate the sources of mineral nutrition, i.e. nitrogen-containing and phosphorus-containing salts. These additives are necessary elements of the yeast nutrition and take part in the build-up of the biomass' cells growing in the course of fermentation.

Conventionally, the concentration of nitrogen in the substrate is from 50 to 600 mg/l and depends on concentration of carbohydrates. In the prior art, mineral salts such as ammonium sulphate, ammophos, or urea are used as the nitrogen nutrition of the yeast for carrying out the alcohol fermentation.

The present inventors have found that the yeast assimilates nitrogen of amino acids faster than nitrogen of mineral salts, which determines rapid development of the yeast culture and high speed of the alcohol fermentation.

The present method for fermentation of carbohydrate substrates of plant origin is characterized in that the amino acids leucine, isoleucine or valine, or a mixture thereof is used as a nitrogen-containing component for the preparation of the carbohydrate substrate in an amount providing a content of the amino nitrogen of from 120 to 420 mg/l of fermentation medium, as defined in the claims. The method may be further characterized by subsequent fermentation of the carbohydrates of the substrate with a specific speed of alcohol fermentation of up to 4.0 1/kg/hour and a yield of C₃-C₅ alcohols in an amount of from 0.65% to 3.1% of ethyl alcohol. The carbohydrate substrate used is beet or cane molasses, acid or enzymatic hydrolysate of starch-containing or cellulose-containing plant materials.

The alcohol yeast, obtained in the fermentation of the molasses' carbohydrates, is condensed to a dry substance content of 5-10%, washed with water during the process of condensation and treated by autolysis at 45-55°C during 24-48 hours. The obtained autolysate with amino nitrogen content of 3000-8000 mg/l containing the amino acids valine, leucine and isoleucine is used as a nitrogen nutrition source for the yeast for fermenting the carbohydrate substrates, as defined in the claims.

Suspended substances of the distillery dreg, after extraction of alcohol, obtained in the fermentation of starch-containing plant materials can be condensed to a dry substance content of 5-10%, followed either by enzymatic hydrolysis of proteins of the distillery dreg free of alcohol at pH 2 - pH 8 and a temperature of 30-60°C using proteolytic enzymatic preparations, such as proteases, including exopeptidases: aminopeptide-aminoacide hydrolases, carboxypeptide-aminoacidohydrolases; and endopeptidases: dipeptidhydrolases and peptide-peptidehydrolases, or by acid hydrolysis of proteins of the distillery dreg free of alcohol at 40-90°C using 0.2-0.5% sulphuric or hydrochloric acid. The amino-acid hydrolysate obtained containing the amino acids valine, leucine and isoleucine with amino nitrogen content of 2000-6000 mg/l can then be used as nitrogen nutrition of the yeast in the fermentation of carbohydrate substrates, as defined in the claims.

Alternatively, combined acid hydrolysis of the cellulose-containing plant material and of the microorganisms' biomass in the ratio of cellulose to biomass of 20:1-100:1 can be carried out. The obtained hydrolysate containing 3-20% of carbohydrates and 50-600 mg/l of amino nitrogen is used for the alcohol fermentation of carbohydrates, as defined in the claims.

Water-soluble substances of the distillery dreg, after extraction of alcohol, can be used for the aerobic cultivation of the yeast; the obtained yeast is condensed to a dry substance content of 5-10% and treated by autolysis at 45-55°C during 24-48 hours. The obtained autolysate containing the amino acids valine, leucine and isoleucine with an amino-nitrogen concentration of 3000-8000 mg/ is used as nitrogen nutrition of the yeast in the fermentation of carbohydrate substrates, as defined in the claims.

The amino acid autolysate of the yeast, containing the amino acids valine, leucine, and isoleucine in an amount providing a content of amino nitrogen in the carbohydrate substrate of from 120 to 420 mg/l, which autolysate has been obtained in the aerobic cultivation of the yeast with pentose-containing distillery dreg, after extraction of alcohol, can be used as nitrogen nutrition in the fermenting of carbohydrate substrates. Pentose-containing distillery dreg, after extraction of alcohol, can be used for the aerobic cultivation of the yeast; the obtained yeast is condensed to a dry substance content of 5-10%, washed with water during the condensation and treated by autolysis at 45-55°C during 24-48 hours. The autolysate thus obtained, containing 3000-8000 mg/l of amino nitrogen, is used as nitrogen nutrition of the yeast in the fermentation of carbohydrate substrates, as defined in the claims.

After extraction of asparagine and ammonium salts, the autolysate of the yeast protein, the acid or enzymatic hydrolysates of the protein of the distillery dreg are used as nitrogen nutrition in the fermentation of the carbohydrate substrates.

Formation of C₃-C₅ alcohols is a result of active functioning of the process of deamination of amino acids in the yeast cells with formation of free ammonia. We have demonstrated that formation of C₃-C₅ alcohols in the process of alcohol fermentation is determined by assimilation of nitrogen from the amino acids valine, leucine, and isoleucine by the growing cells. The yield of C₃-C₅ alcohols reached 3.1% of ethanol when pure valine, isoleucine, and leucine were the sole source of nitrogen nutrition of the yeast in the present fermentation process. Moreover, the maximum formation of C₃-C₅ alcohols in the present process of alcohol fermentation occurred at pH=6.0 of the medium and at 38°C (standard conditions of alcohol fermentation pH 4.5-5.5; temperature 28-34°C).

It has been found that presence in the substrate of asparagine and ammonium ions, in addition to the amino acids valine, leucine, and isoleucine, inhibits formation of C₃-C₅ alcohols. Other amino acids do not inhibit the process of C₃-C₅ alcohols formation. The inhibition constant for the system leucine - ammonium sulphate is 750 mg/l, leucine - asparagine 730 mg/l, valine - asparagine 650 mg/l.

When amino acid autolysate of the yeast was used, the maximum yield of C₃-C₅ alcohols reached 1.1-2.1% of ethanol, and when the amino acid protein hydrolysate of the distiller's grains were used, the maximum yield of C₃-C₅ alcohols was 0.65-0.8% of ethanol. The relatively low yield of C₃-C₅ alcohols, when using the yeast autolysate or distillery dreg protein hydrolysate, is a result of the presence of asparagine.

Wastes of the production of ethyl alcohol from carbohydrate substrates are: biomass of the alcohol yeast, which is increased during the process of fermentation; non-fermentable soluble organic components of the substrate, such as pentose sugar, organic acids, hexose and ethanol residues; non-soluble protein components of grain, etc.

There are known methods for utilisation of said wastes for producing baking yeast, fodder protein and amino acid products.

Biomass of the alcohol yeast or the yeast obtained in aerobic cultivation using non-fermentable organic components of the substrate can be used for obtaining amino acids by known methods of autolysis. Non-soluble protein waste of the ethyl alcohol production can be also used for obtaining amino acids by known methods of enzymatic or acid hydrolysis of protein.

Extraction of ammonia and asparagine from the amino acid mixture by known methods of ion exchange can be used in order to increase the yield of C₃-C₅ alcohols in terms of ethanol, when using yeast autolysate or hydrolysate, and acid or enzymatic hydrolysate of the distillery dreg as nitrogen nutrition of the yeast in the process of carbohydrate substrates fermentation.

The total content of C₃-C₅ alcohols increases from 0.8-2.1% to 2.2-2.95% of ethanol when yeast autolysate free of ammonia and asparagines, or yeast hydrolysate and acid or enzymatic hydrolysate of the distillery dreg protein is used as nitrogen nutrition in the process of carbohydrate substrates fermentation in the production of ethanol.

In the processes of acetone and glycerine biosynthesis it is proposed to use a method similar to that used according to the invention in the biosynthesis of ethanol to increase the yield of C₃-C₅ alcohols, that is to use at the stage of carbohydrate substrate preparation as a nitrogen-containing component the amino acids leucine, isoleucine, valine or mixtures of said acids, including those extracted from the yeast or distillery dreg protein.

To increase the extent of biomass conversion in the synthesis of hydrocarbons and oxygen-containing compounds it is proposed to use for methane biosynthesis distillery dreg, after extraction of alcohol, containing excess amino acids produced in the autolysis or hydrolysis of the yeast. Methane should be obtained under anaerobe conditions using methane-producing bacteria.

To increase the yield of C₁-C₅ alcohols it is proposed to process glycerine (glycerol) obtained in the biosynthesis and from the saponification of fats, into n-propanol. To increase the yield of higher alcohols it is proposede to use vegetable and animal fats in addition to glycerine obtained as a result of biosynthesis in the processing of said glycerine into n-propanol by hydrogenation. The process of hydrogenation of the mixture of glycerine and vegetable and/or animal fats into a mixture of n-propyl alcohol, higher C₆-C₂₀ alcohols and C₆ and higher hydrocarbons can be performed in the presence of copper-chromium, zinc-chromium, nickel-chromium catalysts at 300±100°C and a pressure of 10-30 MPa by hydrogen obtained from biomass. This process can be also carried out in the presence of catalysts comprising precious metals, such as Pt, Pd, Re, Ru, Rh at 200±50°C and a pressure of 5-20 MPa.

It is proposed to condensate C₁-C₅ alcohols produced by the present method into higher alcohols, esters and acids. The condensation can be carried out at a temperature of 100-400°C and a pressure of 0.1-10 MPa in the presence of alcoholates of alkali metals or caustic alkali.

To increase the extent of biomass conversion in the synthesis of hydrocarbons and oxygen-containing compounds it is proposed to use carbon dioxide, obtained in the enzymatic processing of biomass or a mixture of carbon dioxide and hydrogen, for producing methanol. It is also proposed to use hydrogen obtained from biomass and/or from the water obtained in the processing of alcohols obtained in biosynthesis. Water conversion can be carried out by means of known methods. Synthesis of methanol using the raw materials originating from biomass can be carried out at a temperature of 350-450°C in the presence of ZnO-Cr₂O₃ catalyst or at a pressure 4-6 MPa and a temperature of 220-280°C in the presence of CuO-ZnO-Al₂O₃ (Cr₂O₃). Methanol obtained from carbon dioxide is then directed to the processes for production of higher hydrocarbons and oxygen-containing compounds.

To increase the extent of biomass conversion in the synthesis of hydrocarbons and oxygen-containing compounds we propose to use carbon dioxide obtained in the enzymatic processing of biomass for producing carbon oxide. Besides carbon dioxide aforesaid production can use gaseous products of biomass pyrolysis including wood, lignin, turf, solid waste of grain production and wood processing, and lignin obtained in the hydrolysis of cellulose-containing raw material. This process can be carried out in industrial gas generators with boiling or pseudo-liquefied layer of solid particles or in gas generators of other types. The source gas is a mixture of carbon dioxide and oxygen. The reaction temperature is 1000-1500°C. If needed the process of carbon dioxide production can be carried out at a pressure 2-6 MPa. Carbon oxide obtained from the biologic raw material is subsequently mixed with hydrogen obtained from biomass and/or with hydrogen obtained from the water obtained in dehydration of alcohols obtained in biosynthesis or from the water obtained in condensation of aldehydes obtained from said alcohols. Conversion of the water is carried out by known methods. This gas mixture is then used for synthesis of hydrocarbons, including higher alcohols, and other oxygen-containing compounds.

For the oxidation of alcohols into aldehydes it is proposed to use carbon dioxide obtained in the process of biosynthesis. Oxidation of alcohols into aldehydes is carried out at a temperature of 450-650°C and a pressure of 0.05 MPa in the presence of a silver catalyst Ag-Al₂O₃. In contrast to the known processes the steam-gaseous mixture of C₁-C₅ alcohols and carbon dioxide heated to 180-200°C is directed to the oxidation. The use of this mixture gives a possibility to use for the oxidation oxygen or a mixture of oxygen and carbon dioxide. We propose to carry out the condensation of aldehydes, obtained from lower alcohols, with furfural in the alkali medium at 0-10°C. It is further proposed to hydrogenate unsaturated aldehydes obtained in croton condensation of aldehydes, which are obtained in oxidation of lower C₁-C₅ alcohols, and also unsaturated aldehydes, obtained in condensation of furfural with lower C₁-C₅ aldehydes, by hydrogen obtained from biomass and/or by hydrogen obtained from the water obtained in oxidation of alcohols or in condensation of aldehydes. Conversion of the water is carried out by known methods.

For oxidation of aldehydes into fatty acids we propose to use carbon dioxide obtained in biosynthesis. Oxidation of aldehydes into fatty acids is carried out at a temperature of 50-250°C and a pressure of 0.05-0.5 MPa in the presence of a manganese acetate catalyst. In contrast to the known methods the steam-gas mixture of aldehydes and carbon dioxide heated to 50-150°C is supplied to the oxidation. Utilization of said mixture gives a possibility to use for the oxidation oxygen or a mixture of oxygen and carbon dioxide.

For etherification of the fatty acids obtained by the present method we propose to use a mixture of C₁-C₅ alcohols produced by the present method, or to use a mixture of unsaturated C₂-C₅ hydrocarbons obtained from said alcohols. Furthermore, to increase the yield of higher esters we propose to use at the stage of etherification fatty acids, obtained by the present method, C₂-C₆ fatty acids obtained by biosynthesis, and also acids obtained in the saponification of the fats and extracted from tall oil. We propose to perform the etherification in the gas phase at a temperature of 100-200°C and a pressure of 0.5-2.5 MPa in the presence of sulphocationite catalyst or in the liquid phase at a temperature of 50-200°C and a pressure of 0.1-0.5 MPa in the presence of non-organic acids as a catalyst.

For obtaining acetals and ketals it is proposed to use acetaldehyde, acetone, glycerine, and a mixture of C₃-C₅ alcohols produced by the present method, acetaldehyde obtained in oxidation of ethanol produced by biochemical method, and formaldehyde obtained in oxidation of methanol synthesized from carbon dioxide produced by biochemical method. It is proposed to carry out the process of acetals and ketals production in the liquid phase at a temperature of 0-50°C and a pressure of 0.1-0.5 MPa using hydrochloric or sulphuric acids or salts of these acids as a catalyst.

For producing synthesis gas wastes of the grain production, vegetable oils, wood processing, including pulp production and production of wood coal, and also by-products and wastes obtained in biosynthesis of C₁-C₅ alcohols, glycerine, acetaldehyde, acetone, C₂-C₆ acids, and by-products and wastes obtained in chemical processing of the aforesaid oxygen-containing compounds can be used. For the production of synthesis gas it is also proposed to use biogas obtained in the fermentation of the various types of biomass and carbon dioxide obtained at the fermentation stage of the same production or carbon dioxide obtained in the biosynthesis of other bio-products. For producing synthesis gas, besides carbon dioxide obtained in biosynthesis, it is also possible to use the gases and resins obtained in the pyrolysis of wood, furfural, turpentine, colophony, tall oil, fusel oils, vegetable oils and wastes of the production of aforesaid products. The process of synthesis gas production is carried out at a temperature of 800-1100°C and a pressure of 0.1-3 MPa in the presence of an Al₂O₃ supported NiO catalyst or at 1450-1550°C and a pressure of 2-10 MPa without a catalyst. It is proposed to use synthesis gas obtained by the present method for producing hydrocarbons and oxygen-containing compounds by the Fisher-Tropsch method and by processes based on the reaction of hydroformylation.

It is proposed to carry out the production of hydrocarbons by the Fisher-Tropsch method from synthesis gas, obtained by the present method, at a temperature of 200-350°C and a pressure of 2.0-2.5 MPa in the presence of ferrous catalyst promoted by oxides of alkali metals or at 170-200°C and a pressure of 0.1-1.0 MPa in the presence of cobalt-thorium-magnesium catalyst. The process of production of the oxygen-containing compounds by the Fisher-Tropsch method from synthesis gas obtained by the method should be carried out at a temperature of 180-250°C and a pressure of 1.0-3.5 MPa in the presence of a ferrous-copper catalyst promoted by oxides of aluminium, calcium, zinc, magnesium, and alkaline agents, such as compounds of alkaline metals, which when dissolved in water produce an alkaline reaction.

For producing unsaturated hydrocarbons, which are subsequently directed to hydroformylation or alkylation, it is proposed to dehydrate the mixture of C₂-C₆ alcohols, obtained in biosynthesis, and/or glycerine, as well as the glycerine obtained by saponification of fats. Dehydration is carried out at a temperature of 200-400°C and a pressure of 0.1-3 MPa in the presence of an Al₂O₃ catalyst. The mixture of alcohols and/or glycerine can be also dehydrated by heating with sulphuric acid.

It is proposed to alkylate unsaturated hydrocarbons obtained in dehydration of lower C₂-C₅ alcohols by using isobutane and isopentane obtained from the corresponding iso-alcohols, and also using terpenes, which have previously been heated to a temperature of 200±50°C. The result of alkylation, which is carried out at 0-10° and a pressure of 0.5-1 MPa in the presence of 90-100% sulphuric acid as a catalyst, is the obtaining of the mixture of C₆-C₁₅ hydrocarbons. The alkylation can be also carried out in the presence of an AlCl₃ catalyst at a temperature of 50-60°C and a pressure of 1-2 MPa.

It is proposed to perform the process of hydroformylation of unsaturated hydrocarbons obtained in dehydration of lower C₂-C₅ alcohols using synthesis gas, obtained from biomass, at a temperature of 160±20°C and a pressure of 30±10 MPa in the presence of cobalt carbonyl catalyst; or at a temperature of 175±25°C and a pressure fo 7.5±2.5 MPa in the presence of a cobalt catalyst modified by phosphorus compounds; or at 90±10°C and a pressure of 2±1 MPa in the presence of a cobalt-rhodium catalyst.

It is proposed to hydrogenate the aldehydes, obtained in hydroformylation of unsaturated hydrocarbons, and/or acrolein, obtained in dehydration of glycerine into saturated alcohols, by hydrogen obtained from biomass and/or by hydrogen produced from the water obtained in dehydration of alcohols obtained from biosynthesis. Conversion of the water is carried out by known methods. It is proposed to hydrogenate saturated and unsaturated aldehydes into saturated alcohols at a temperature of 50-150°C and a pressure of 1-2 MPa in the presence of an Al₂O₃ supported NiO catalyst or at a temperature of 200-250°C and a pressure of 5-20 MPa in the presence of a CuO-Cr₂O₃ catalyst.

Thus, the present method for production of higher hydrocarbons, including oxygen-containing compounds, from biomass offers a solution to the following problems:
- To produce higher oxygen-containing compounds and/or non oxygen-containing hydrocarbons, including those having four and more carbon atoms in the molecule, from biomass using the raw material obtained by biochemical methods;
- To considerably increase the yield of C₃-C₅ alcohols in the process of their biosynthesis by fermenting the carbohydrate substrates;
- To increase by 1.5-2.0 times the productivity of the fermentation stage for the technology of C₁-C₅ alcohols production;
- To utilize the protein-containing waste and other bio components of the distillery dreg after extraction of alcohol within the frame of the technology for C₁-C₅ alcohols production, including for the purpose of methane production;
- To utilize in the production of hydrocarbons, including oxygen-containing compounds, carbon dioxide obtained in the biosynthesis of C₁-C₅ alcohols, and also carbon dioxide obtained in the biosynthesis of other lower hydrocarbons;
- To utilize in the production of hydrocarbons, including oxygen-containing compounds, fats, glycerine obtained in the saponification of fats, furfural obtained in hydrolysis of pentosane-containing raw material, C₂-C₆ fatty acids obtained by biosynthesis, acids obtained in the saponification of fats and extracted from tall oil, resins, turpentine, colophony and tall oil obtained in the wood processing;
- To increase the rate of the direct use of biomass for synthesis of higher alcohols and other oxygen-containing compounds, and also higher hydrocarbons;
- To use hydrocarbons, including oxygen-containing compounds, obtained from biomass by the present method as a component for motor fuels.

### EXAMPLE 1

Crushed wheat grain was mixed with water in a ratio of 1:3.5. Enzymatic hydrolysis of the grain starch was accomplished using in the first stage thermostable amylase Zy-majunt-340C (pH 6.5, 90°C, consumption 0.25 ml per 1 kg of the grain starch), and in the second stage glycoamylase Glucozym L-400C (pH 5.0, 60°C, consumption 0.8 ml per 1 kg of the grain starch). Industrial enzymes produced by Ende Industries Inc., USA have been used. As a result of enzymatic hydrolysis the concentration of carbohydrates in the substrate reached 16%. To the substrate were added: superphosphate in an amount providing a content of P₂O₅ of 200 mg/l, and the amino acid leucine in an amount of 4000 mg/l (amino nitrogen 420 mg/l). Starter yeast biomass S. cerevisiae was introduced to the substrate at a concentration of 5 g/l. The fermentation was carried out at a temperature of 38°C and a pH of 6.0.

The speed of fermentation was 3.0 1/kg*h, the ethanol concentration at the end of fermentation was 8.9% by vol., and the isopentanol concentration 2300 mg/l or 3.1% of the volume of ethanol.

### EXAMPLE 2

Crushed wheat grain was mixed with water in a ratio of 1:3.5. Enzymatic hydrolysis of the grain starch was carried out using in the first stage thermostable amylase Zy-majunt-340C (pH 6.5, 90°C, consumption 0.25 ml per 1 kg of the grain starch), and in the second stage glucoamylase Glucozym L-400C (pH 5.0; 60°C, consumption 0.8 ml per 1 kg of the grain starch). Industrial enzymes produced by Ende Industries Inc., USA were used. As a result of the enzymatic hydrolysis the concentration of carbohydrates in the substrate reached 16%. To the substrate were added: superphosphate in an amount providing a P₂O₅ content of 200 mg/l, and the amino acid valine in an amount of 3000 mg/l (amino nitrogen 360 mg/l). The yeast starter biomass S. cerevisiae was introduced to the substrate in an amount of 5 g/l. The fermentation was carried out at a temperature of 38°C and a pH of 6.0.

The fermentation speed was 2.8 1/kg*h, the ethanol concentration at the end of fermentation was 8.9% vol., and the isobutanol concentration 1810 mg/l or 2.5% of the ethanol volume.

### EXAMPLE 3

Crushed wheat grain was mixed with water in a ratio of 1:3.5. Enzymatic hydrolysis of the grain starch was carried out using in the first stage thermostable amylase Zy-majunt-340C (pH 6.5, 90°C, consumption 0.25 ml per 1 kg of the grain starch) and in the second stage glucoamylase Glucozym L-400C (pH 5.0, 60°C, consumption 0.8 ml per 1 kg of the grain starch). Industrial enzymes produced by Ende Industries Inc., USA were used. As a result of the enzymatic hydrolysis the carbohydrate concentration in the substrate reached 16%. To the substrate were added: superphosphate in an amount providing a P₂O₅ content of 200 mg/l, and the amino acid isoleucine in an amount of 4000 mg/l (amino nitrogen 420 mg/l). The yeast starter biomass S. cerevisiae was introduced to the substrate in the concentration of 5 g/l. The fermentation was carried out at a temperature of 38°C and a pH of 6.0. The speed of fermentation was 3.0 1/kg*h, the ethanol concentration at the end of fermentation reached 8.9%, the isopenthanol concentration 2120 mg/l or 2.8% of the ethanol volume.

### EXAMPLE 4

Crushed wheat grain was mixed with water in a ratio of 1:3.5. Enzymatic hydrolysis of the grain starch was carried out using in the first stage thermo-stable amylase Zy-majunt-340C (pH 6.5, 90°C, consumption 0.25 ml per 1 kg of the grain starch) and in the second stage glucoamylase Glucozym L-400C (pH 5.0; 60°C, consumption 0.8 ml per 1 kg of the grain starch). Industrial enzymes produced by Ende Industries Inc., USA have been used. As a result of the enzymatic hydrolysis the carbohydrates concentration in the substrate has reached 16%. Superphosphate was added to the substrate in an amount providing a P₂O₅ content of 200 mg/l, the amino acid leucine was added in an amount of 1000 mg/l, the amino acid isoleucine in the amount of 1000 mg/l, and the amino acid valine in the amount 1500 mg/l. The yeast starter biomass S. cerevisiae was added to the substrate in the amount of 5 g/l. The fermentation was carried out at a temperature of 38°C and a pH=6.0.

The fermentation speed was 3.5 1/kg*h, the ethanol concentration at the end of the fermentation was 8.8% vol., the isopentanols concentration 1290 mg/l, and the isobutanol concentration 910 mg/l, or the total content of C₄-C₅ alcohols was 3% of the ethanol volume.

### EXAMPLE 5

Crushed wheat grain was mixed with water in a ratio of 1:3.5. Enzymatic hydrolysis of the grain starch was carried out using in the first stage thermostable amylase Zy-majunt-340C (pH 6.5, 90°C, consumption 0.25 ml per 1 kg of the grain starch) and in the second stage glucoamylase Glucozym L-400C (pH 5.0; 60°C; consumption 0.8 ml per 1 kg of the grain starch). Industrial enzymes produced by Ende Industries Inc., USA have been used. As a result of the enzymatic hydrolysis the carbohydrate concentration in the substrate reached 16%. To the substrate were added: superphosphate in an amount providing a P₂O₅ content of 200 mg/l, and liquid autolysate of the alcohol yeast in an amount of 50 ml/l (amino nitrogen 320 mg/). The yeast starter biomass S. cerevisiae was added to the substrate in an amount of 5 g/l. The fermentation was carried out at a temperature of 38°C and a pH 6.0. The speed of the fermentation was 4.0 1/kg*h, the ethanol concentration at the end of fermentation was 8.8% vol., the isopentanols concentration 480 mg/l, and the isobutanol concentration 270 mg/l. The total content of C₃-C₅ alcohols was 1.1% of the volume of ethanol.

### EXAMPLE 6

Beet molasses with a saccharose concentration of 46% was diluted with water to a saccharose concentration of 18%, acidified by sulphuric acid to pH 5.5, then the alcohol yeast autolysate was added in an amount of 50 ml/l (350 mg/l of the amino nitrogen) and the yeast starter biomass S. cerevisiae in the amount of 5 g/l. The fermentation was carried out at a temperature of 38°C and pH 5.5. The fermentation speed was 3.8 1/kg*h, the ethanol concentration at the end of fermentation was 8.6% vol., the isopentanols concentration 490 mg/l, the isobutanol concentration 290 mg/l, and the total content of C₃-C₅ alcohols was 1.1% of the volume of ethanol while the concentration of the alcohol yeast biomass was 6.2 g/l.

The alcohol yeast was separated from the liquid culture by filtration and washed with water. The obtained yeast was used for preparation of the suspension with a dry substance content of 12%. Autolysis of the yeast was carried out and the suspension was let to stand in a thermostat at a temperature of 48°C for 36 hours. The content of amino nitrogen in the autolysate obtained was 7000 mg/l, and the amount of the obtained autolysate was 55 ml/l of the medium. The obtained autolysate was used for preparing the source medium for fermentation of the molasses substrate.

### EXAMPLE 7

Sugar cane molasses with a saccharose concentration of 46% was diluted with water to a saccharose concentration of 18%, acidified by sulphuric acid to pH 5.5, and then the alcohol yeast autolysate was added in an amount of 60 ml/l (370 mg/l of the amino nitrogen) and the yeast starter biomass S. cerevisiae in the amount of 5 g/l. The fermentation was carried out at a temperature of 38°C and pH 5.5. The fermentation speed was 4.0 1/kg*h, the ethanol concentration at the end of fermentation was 8.7% vol., the isopentanols concentration 470 mg/l, isobutanol concentration 290 mg/l, and the total content of C₃-C₅ alcohols was 1.2% of the volume of ethanol.

Ethanol, C₃-C₅ alcohols and other volatile components were distilled off from the after-fermentation culture liquid (mash). To the alcohol-free mash (distillery dreg) nitrogen and phosphorus mineral salts were added, and aerobic cultivation of the yeast Candida tropicalis was carried out. As a result of the cultivation, a yeast suspension with a biomass concentration of 15 g/l was obtained. The yeast was separated from the culture liquid by filtration, washed with water and treated by autolysis as described in Example 6. The amino nitrogen content in the obtained autolysate was 6500 mg/l, the amount of autolysate 125 mg/l of the medium. The obtained autolysate was used for preparing the source medium for fermenting the molasses substrate.

### EXAMPLE 8

Beet molasses with a saccharose concentration of 46% was diluted with water to a saccharose concentration of 18%, acidified by sulphuric acid to pH 5.5, then the acid hydrolysate of the yeast in an amount of 120 ml/l (350 mg/l of the amino nitrogen) and the yeast starter biomass S. cerevisiae in the amount of 5 g/l were added. The fermentation was carried out at a temperature of 38°C and pH 5.5. The fermentation speed was 3.4 1/kg*h, the ethanol concentration at the end of the fermentation was 8.7% vol., the isopentanols concentration 460 mg/l, isobutanol concentration 290 mg/l, and the total content of C₃-C₅ alcohols was 1.2% of the volume of ethanol.

Ethanol, C₃-C₅ alcohols and other volatile components were distilled off from the after-fermentation culture liquid (mash). To the alcohol-free mash (distillery dreg) nitrogen and phosphorus mineral salts were added, and aerobic cultivation of the yeast Candida tropicalis was carried out. As a result of the cultivation a yeast suspension with a biomass concentration of 15 g/l was obtained. The yeast was separated from the culture liquid by filtration, washed with water and a biomass suspension with a dry substance content of 6% was prepared. Hydrolysis of the suspension was carried out in the presence of 4N HCl at 100°C for 12 hours. The amino nitrogen content in the obtained hydolysate was 3100 mg/l, the amount of hydrolysate 240 ml/l of the medium. The obtained acid hydrolysate was used for preparing the source medium for fermenting the molasses substrate.

### EXAMPLE 9

Chopped spruce wood (cellulose-containing plant material) was treated by acid hydrolysis at a temperature of 180°C, a sulphuric acid concentration of 0.5%, a ratio of water to wood of 12:1, and during a time of 1.5 hours. The hydrolysate of the wood was neutralized with lime to a pH of 4.5, and separated from lignin and gypsum residues. To the carbohydrate substrate obtained, with a hexose sugar concentration of 3.2% and pentose sugar concentration of 0.8%, superphosphate was added in an amount of P₂O₅ 120 mg/l, the yeast autolysate in an amount of 40 ml/l of substrate (120 mg/l of amino nitrogen), and 5 g/l of the starter yeast biomass S. cerevisiae. The fermentation was carried out at a temperature of 38°C and pH 5.5. The fermentation speed was 3.7 1/kg*h, the ethanol concentration at the end of the fermentation was 1.5% vol., concentration of isopentanols was 170 mg/l, concentration of isobutanol was 90 mg/l, and the total content of C₃-C₅ alcohols was 2.1% of the volume of ethanol.

Ethanol, C₃-C₅ alcohols and other volatile components were distilled off from the after-fermentation culture liquid (mash). Nitrogen and phosphorous mineral salts were added to the alcohol-free pentose-containing distillery dreg and aerobic cultivation of Candida tropicalis yeast was carried out. As a result of the cultivation a yeast suspension with a biomass concentration of 6 g/l was obtained. The yeast was separated from the culture liquid by filtration, washed with water, and a suspension with a dry substance concentration 12% was prepared. Autolysis of the yeast was carried out by treating the suspension in the thermostat at 48°C for 36 hours. The content of amino nitrogen in the obtained autolysate was 7100 mg/l, the amount of the autolysate was 50 ml/l of the medium. The obtained autolysate was used for fermenting the wood hydrolysate.

### EXAMPLE 10

Chopped spruce wood (cellulose containing plant material) was treated by acid hydrolysis at a temperature of 180°C, a sulphuric acid concentration of 0.5%, a ratio of water to wood of 12:1, and for a time of 1.5 hours. The wood hydrolysate was neutralized by lime to a pH = 4.5, separated from lignin and gypsum residues. To the carbohydrate substrate obtained, having a concentration of hexose and pentose sugars of 3.2% and 0.8%, respectively, were added: superphosphate in an amount of P₂O₅ of 120 mg/l, yeast autolysate, purified of ammonia and asparagines by known methods of ion exchange, in the amount of 40 ml/l of substrate (120 mg/l of amino nitrogen), and starter yeast biomass S. cerevisiae in a concentration of 5 g/l. The fermentation was carried out at 38°C and pH=6. The speed of the fermentation was 4.0 1/kg*h, ethanol concentration at the end of the fermentation was 1.5% vol., the isopentanols and isobutanol concentrations were 210 mg/l and 120 mg/l, respectively. The total content of C₃-C₅ alcohols was 2.9% of the volume of ethanol.

Ethanol, C₃-C₅ alcohols and other volatile components were distilled off from the after-fermentation culture liquid (mash). Nitrogen and phosphorous mineral salts were added to the alcohol-free pentose-containing distillery dreg, and aerobic cultivation of the yeast Candida tropicalis was carried out. As a result of the cultivation a yeast suspension having a biomass concentration of 6 g/l was obtained. The yeast was separated from the culture liquid by filtration, washed with water with subsequent preparation of the biomass suspension with 12% content of dry substance. Autolysis of the yeast was accomplished by letting the suspension stand at a temperature of 48°C during 36 hours. The amino nitrogen concentration in the obtained autolysate was 8000 mg/l, the amount of the obtained autolysate was 50 ml/l of the medium. The amino acid autolysate thus obtained was treated by ion exchange to extract ammonia nitrogen and asparagines; after that the mixture of amino acids without asparagines and ammonia nitrogen was used as nitrogen nutrition for fermentation of the wood hydrolysate.

### EXAMPLE 11

Chopped spruce wood (cellulose-containing plant material) was used together with the yeast biomass in a ratio of 50:1 and treated by acid hydrolysis at a temperature of 180°C, with a sulphuric acid concentration of 0.5%, a ratio of water to wood of 12:1, and for a period of time of 1.5 hours. The hydrolysate was then neutralized by lime to a pH = 4.5, separated from lignin and gypsum residues. The obtained carbohydrate substrate having a concentration of hexose and pentose sugars of 3.2% and 0.8% respectively, was subsequently added with superphosphate in an amount of P₂O₅=120 mg/l. The content of amino nitrogen in the substrate obtained in the yeast protein hydrolysis was 130 mg/l. Disseminating yeast biomass S. cerevisiae was then supplied to the hydrolysate in the amount of 5 g/l. The fermentation was carried out at a temperature of 38°C and a pH = 5.5. The speed of fermentation was 3.5 1/kg*h, ethanol concentration at the end of the fermentation was 1.5% vol., isopentanols and isobutanol concentrations were 140 mg/l and 80 mg/l, respectively. The total content of C₃-C₅ alcohols was 1.8% of the volume of ethanol.

Ethanol, C₃-C₅ alcohols and other volatile components were distilled off from the after-fermentation culture liquid (mash). Nitrogen and phosphorous mineral salts were added to the alcohol-free pentose-containing distillery dreg, and aerobic cultivation of the yeast Candida tropicalis was carried out. As a result of the cultivation a yeast suspension having a biomass concentration of 6 g/l was obtained. The yeast was separated from the culture liquid by filtration, washed with water and dried. The yield of the yeast biomass in terms of the consumed wood was 48 g/kg. The obtained yeast biomass was used for the acid hydrolysis of the wood.

Carbon dioxide obtained in the biosynthesis of alcohols was mixed with oxygen and directed to a gas generator. Granulated lignin obtained in the hydrolysis of wood was supplied to the same gas generator simultaneously with the source gas. During the granulation lignin was added with resin, obtained in the pyrolysis of wood, colophony, and wastes obtained in the processing of turpentine, tall oil, fusel and vegetable oils. The process of carbon oxide production was carried out at a temperature of 1000-1500°C.

Carbon oxide thus obtained from the biological raw material was mixed with hydrogen obtained by electrolysis of water. This gas mixture was then used for synthesis of higher alcohols based on the reaction of hydroformylation, and also for producing hydrocarbons and oxygen-containing compounds by the Fisher-Tropsch method.

Obtaining of hydrocarbons by the Fisher-Tropsch method was carried out as follows. Synthesis gas obtained by the present method with a ratio of components CO:H₂=1:0.75 at a temperature of 190-230°C and a pressure of 2-2.5 MPa was directed through the reactor filled with a catalyst, comprising the following: 97% Fe₃O₄ + 2.5% Al₂O₃ + 0.5% K₂O. The yield of the products per 1 m³ was the following: the liquid 140-150 g + the gas 30-40 g. The gas comprised C₁-C₄ hydrocarbons; the liquid was boiling away in the interval 30-400°C. 40-50% of the liquid are non-oxygen-containing hydrocarbons and 50-60% of the liquid are oxygen-containing compounds, with prevailing C₆ and higher alcohols. The process can also be performed at a temperature of 180-220°C and a pressure of 2.5-3 MPa in the presence of a catalyst comprising Fe:Cu=10:1 promoted by oxides of aluminum, calcium, zinc, magnesium, manganese and alkali agents. These parameters of the process allow using synthesis gas obtained by the present method and having a ratio of the components CO:H₂=1:1.25. In this case the yield of the products per 1 m³ is: the liquid 160-170 g + gas 20-30 g.

### EXAMPLE 12

Beet molasses having a saccharose concentration of 46% was diluted with water to a saccharose concentration of 18%, acidified with sulphuric acid to a pH of 5.5, and the yeast acid hydrolysate after ion-exchange was added in an amount of 120 ml/l (360 mg/l of amino nitrogen), and the yeast starter biomass S. cerevisiae in an amount of 5 g/l. The fermentation was carried out at a temperature of 38°C and a pH of 5.5. The speed of the fermentation was 3.6 1/kg*h, the ethanol concentration at the end of the fermentation was 8.7% vol.; isopentanols and isobutanol concentrations were 1000 mg/l and 490 mg/l, respectively. The total content of C₃-C₅ alcohols was 2.2% of the volume of ethanol.

Ethanol, C₃-C₅ alcohols and other volatile components were distilled from the after-fermentation culture liquid (mash). Nitrogen and phosphorus mineral salts were added to the alcohol-free mash (distillery dreg) and aerobic cultivation of the yeast Candida tropicalis was carried out. As a result of the cultivation a yeast suspension having a biomass concentration of 15 g/l was obtained. The yeast was separated from the culture liquid by filtration, washed with water and treated by autolysis, and a biomass suspension with a dry substance concentration of 6% was prepared. Hydrolysis of the suspension was carried out with 4N HCl at 100°C for 12 hours. The amino nitrogen content in the obtained hydrolysate was 3100 mg/l, the ammonia nitrogen content was 420 mg/l, the amount of hydrolysate 240 ml/l of the medium. The obtained acid hydrolysate was treated by ion exchange on a cationic exchanger to extract ammonia nitrogen. The obtained mixture of amino acids free of asparagine and ammonia nitrogen was used in the preparation of the source medium for fermentation of the molasses substrate.

The wastes obtained in the acid hydrolysis of the biomass, extracted after cultivation of the yeast, are mixed with a surplus of amino acids left after preparation of the source medium for fermentation of the molasses substrate, diluted with cultural liquid to the concentration 50 g/1, and directed to the methane tank, containing methane-producing bacteria Methanobacterium thermoautotropicum, for producing methane. Production of methane in the form of biogas was carried out under strict anaerobe conditions. The productivity of the methane tank was 11 of methane per 2 1 of nutritious medium per 24 hours. Biogas thus obtained was used as a base for producing synthesis-gas.

### EXAMPLE 13

Crushed wheat grain was mixed with water in the ratio of 1:3.5. Enzymatic hydrolysis of the grain starch was carried out using In the first stage thermostable amylase Zy-majunt-340C (pH 6.5, 90°C, consumption 0.25 ml per 1 kg of the grain starch) and In the second stage glucoamylase Glucozym L-400C (pH 5.0; 60°C; consumption 0.8 ml per 1 kg of the grain starch). Industrial enzymes produced by Ende Industries Inc., USA have been used. As a result of the enzymatic hydrolysis the carbohydrate concentration in the substrate reached 16%. To the substrate were then added superphosphate in an amount providing a P₂O₅ content of 200 mg/l, amino acid leucine in the amount of 2000 mg/l and amino acid valine in the amount 1500 mg/l (amino nitrogen content of 390 mg/l). The yeast starter biomass S. cerevisiae was added to the substrate in an amount of 5 g/l. The fermentation was carried out at a temperature of 38°C and pH=6.0. The speed of fermentation was 3.5 1/kg*h, ethanol concentration at the end of the fermentation was 8.8% vol., isopentanol concentration 1250 mg/l, and isobutanol concentration 910 mg/l. Total content of C₄-C₅ alcohols was 2.95% of the volume of ethanol. Ethanol, C₃-C₅ alcohols and other volatile components were distilled off from the after-fermentation culture liquid (mash).

Carbon dioxide obtained in the biosynthesis of alcohols was mixed with methane obtained in biosynthesis and water steam, and directed to the reactor for producing synthesis gas. Conversion of the source mixture was carried out in the presence of a NiO-Al₂O₃ catalyst at 830-850°C. The gas mixture thus obtained had the following composition: CO₂ - 4.8% vol.; CO - 24.7% vol.; H₂ - 68.0% vol.; CH₄ - 2.5% vol. Then the converted gas was cooled down, compressed to 5 MPa and directed to methanol synthesis. Methanol synthesis was carried out at 5 MPa and a temperature of 230-260°C in the presence of CuO-ZnO-Al₂O₃ (Cr₂O₃) catalyst. Methanol obtained from carbon dioxide was then directed to the processes for production of higher hydrocarbons and oxygen-containing compounds.

In another process for obtaining synthesis gas we used, besides carbon dioxide obtained in biosynthesis, gases and resins obtained in pyrolysis of wood, wastes of furfural, turpentine, colophony, and fusel oil. The process for obtaining synthesis gas was carried out at a temperature of 800-1100°C and a pressure of 0.1-3 MPa in the presence of a Al₂O₃ supported NiO catalyst. A gas mixture of the following composition was thus obtained: CO₂ - 4.2-4.6% vol.; CO - 41.5-32.7% vol.; H₂ - 44.8-53.3% vol.; CH₄ - 5.5-5.7% vol.; N₂ - 3.3-4.7% vol. Then the converted gas was cooled down and directed to the production of hydrocarbons by the Fisher-Tropsch method. The process was carried out as follows. Synthesis gas obtained by the present method and having the ratio of the components CO : H₂ = 1 : 1.1-1.7 at 220-330°C and a pressure 2.3-2.5 MPa was directed through the reactor filled with ferrous alloy promoted by oxides (Al₂O₃, K₂O, MgO) catalyst. The yield of the products per 1 m³ was 170-180 g. The obtained product was composed of olefins and paraffins, distillation range of the liquid was 30-400°C, the liquid contained 96% of non oxygen-containing hydrocarbons and 4% of the oxygen-containing compounds, 50% of which were C₄ and higher alcohols.

The process can also be carried out at a temperature of 170-200°C and a pressure of 0.1-1.0 MPa in the presence of cobalt-thorium-magnesium catalyst. These process parameters allow using synthesis gas obtained by the present method and having a ratio of the components CO:H₂=1:1.5. The yield of the products in the process is 170-175 g per 1 m³. The product obtained contained olefins and paraffins, the liquid was distilling in the interval 30-400°C, 99% of the liquid were non oxygen-containing hydrocarbons and 1% oxygen-containing compounds, 70% of which were C₁-C₁₀ alcohols.

For producing synthesis gas we used, besides carbon dioxide obtained in biosynthesis, natural gas comprising, mainly, methane. Conversion of the source mixture is carried out in the presence of NiO-Al₂O₃ catalyst at 830-850°C. Thus, a gas mixture similar in composition to the synthesis gas obtained in the conversion of the biologic raw material was obtained, that is: CO₂-4.5% vol.; CO-22.9% vol.; H₂-70.1% vol.; CH₄-2.4% vol.; SO₂+SO₃ - 0.1% vol. However, presence of sulphur oxides in the mixture requires additional purification of the synthesis gas before it is supplied to the catalyst. After sulphur oxides have been extracted from the gas mixture the converted gas was compressed by a compressor to 5 MPa and directed to methanol synthesis. The synthesis of methanol was carried out at a pressure of 5 MPa and a temperature of 230-260°C in the presence of CuO-ZnO-Al₂O₃ (Cr₂O₃) catalyst. Methanol obtained from biochemical carbon dioxide was then directed to the synthesis of higher hydrocarbons and oxygen-containing compounds, including also etherifying of unsaturated and saturated C₈-C₂₄ acids obtained in the saponification of fats and extracted from tall oil.

### EXAMPLE 14

Crushed wheat grain was mixed with water in ratio 1:3.5. Enzymatic hydrolysis of the grain starch was accomplished using in the first stage thermostable amylase Zymajunt-340C (pH 6.5, 90°C, consumption 0.25 ml per 1 kg of the grain starch), and in the second stage glycoamylase Glucozym L-400C (pH 5.0, 60°C, consumption 0.8 ml per 1 kg of grain). Industrial enzymes produced by Ende Industries Inc., USA have been used. The concentration of carbohydrates in the substrate as a result of enzymatic hydrolysis was 16%. To the substrate were added: superphosphate in the amount providing a content of P₂O₅ of 200 mg/l, the amino acid leucine in an amount of 1000 mg/l, amino acid isoleucine in an amount 1000 mg/l, and amino acid valine in the amount of 1500 mg/l (amino nitrogen content of 390 mg/l). Starter yeast biomass S. cerevisiae was introduced to the substrate at a concentration of 5 g/l. The fermentation was carried out at a temperature of 38°C and a pH of 6.0.

The fermentation speed was 3.5 1/kg*h, ethanol concentration at the end of fermentation was 8.8% by vol., isopentanols concentration 1290 mg/l, and isobutanol concentration 910 mg/l. Total content of C₄-C₅ alcohols was 3% of the volume of ethanol.

Ethanol, C₃-C₅ alcohols and other volatile components were distilled from after-fermentation culture liquid (mash).

Ethanol was separated from C₃-C₅ alcohols and other volatile components and dehydrated in the presence of Al₂O₃ at 300±100°C. Ethylene thus obtained was mixed with synthesis gas, originating from biogas and having a ratio CO:H₂=1:1 and directed to the reactor with cobalt-rhodium catalyst. The temperature in the reactor was kept at 90±10°C and the pressure at 2±1 MPa. Propionic aldehyde thus obtained in the reactor was directed to the reactor containing Ni catalyst and hydrogenated at 150±50°C and a pressure of 1-2 MPa into n-propyl alcohol by the hydrogen obtained from biomass. Besides that, propionic aldehyde can be condensed to isohexene aldehyde, with subsequent hydrogenation into isohexanol in the presence of Ni catalyst by hydrogen obtained from biomass.

Besides that ethylene was also telomerised with methanol at 150±20°C and a pressure 7±3 MPa in the presence of tertbutyl peroxide, which has been used to initialise the reaction, to obtain a mixture of oxygen-containing compounds mainly comprising C₃-C₁₂ alcohols of normal structure.

### EXAMPLE 15

Crushed wheat grain was mixed with water in a ratio of 1:3.5. Enzymatic hydrolysis of the grain starch was accomplished using in the first stage thermostable amylase Zy-majunt-340C (pH 6.5, 90°C, consumption 0.25 ml per 1 kg of the grain starch), and in the second stage glycoamylase Glucozym L-400C (pH 5.0, 60°C, consumption 0.8 ml per 1 kg of grain). Industrial enzymes produced by Ende Industries Inc., USA have been used. The concentration of carbohydrates in the substrate as a result of enzymatic hydrolysis was 16%. To the substrate were added: superphosphate an amount providing a content of P₂O₅ of 200 mg/l, and amino acid hydrolysate obtained in enzymatic hydrolysis of the distillery dreg protein in the amount of 70 ml/l (360 mg/l of amino nitrogen). Starter yeast biomass S. cerevisiae was introduced to the substrate at a concentration of 5 g/l. The fermentation was carried out at a temperature of 38°C and a pH of 6.0.

The speed of fermentation was 3.5 1/kg*h, ethanol concentration at the end of fermentation was 8.8% by vol., isopentanols concentration 260 mg/l, and isobutanol concentration 140 mg/l. The total content of C₃-C₅ alcohols was 0.8% of the volume of ethanol.

Ethanol, C₃-C₅ alcohols and other volatile components were distilled from the after-fermentation culture liquid (mash).

Suspended substances of the distillery dreg, after extraction of alcohol, obtained in the fermentation of carbohydrates yielded in starch hydrolysis were condensed to a dry substance content of 5-10%. After that, enzymatic hydrolysis of proteins of the distillery dreg, after extraction of alcohol, using at the first stage endopeptidase Pepsin 2000 FIP-U/g, EC 3.4.23.1 (pH=2, 36°C; consumption 0.5 g per 1 kg of dry substance of the distillery dreg after extraction of alcohol) and at the second stage exopeptidase Aminopeptidase K EC 3.4.11 (pH=8, 36°C, consumption 0.1 g per 1 kg of dry substance of the distillery dreg after extraction of alcohol) was carried out. The amino acid hydrolysate thus obtained having a concentration of amino nitrogen of 2000-6000 mg/l was used as nitrogen nutrition of the yeast in fermenting carbohydrate substrates.

Propyl and isopropyl alcohols were separated from C₂-C₅ alcohols and other volatile components and dehydrated with Al₂O₃ catalyst at 300±50°C. Propylene obtained in the dehydration was mixed with synthesis gas, produced from biogas and having a ratio CO:H₂=1:1, and directed to the reactor with cobalt-rhodium catalyst. The temperature in the reactor was kept at 90±10°C and pressure at 2±1 MPa. Butyl and iso-butyl aldehydes obtained in the reactor were transferred to the reactor with Ni catalyst, where these were hydrogenated at a temperature of 150±50°C and a pressure of 1-2 MPa, using the hydrogen obtained from biomass, into butyl and isobutyl alcohols.

Furthermore, butyl aldehyde can be first condensed into isooctene aldehydes and hydrogenated with Ni-catalyst by hydrogen obtained from biomass into isooctanols.

Besides that propylene was mixed with carbon oxide, obtained from carbon dioxide obtained at the stage of alcohols biosynthesis, and water in the ratio of 1:3:2 in the presence of a complex catalyst comprising ferrous pentacarbonyl, water and triethylamine at 100±10°C and a pressure 1-2 MPa to obtain n-butyl alcohol.

### EXAMPLE 16

Crushed wheat grain was mixed with water in a ratio of 1:3.5. Enzymatic hydrolysis of the grain starch was accomplished using in the first stage thermostable amylase Zy-majunt-340C (pH 6.5, 90°C, consumption 0.25 ml per 1 kg of the grain starch), and in the second stage glycoamylase Glucozym L-400C (pH 5.0, 60°C, consumption 0.8 ml per 1 kg of the grain starch). Industrial enzymes produced by Ende Industries Inc., USA have been used. The concentration of carbohydrates in the substrate as a result of enzymatic hydrolysis was 16%. To the substrate were added: superphosphate in the amount providing a content of P₂O₅ of 200 mg/l, and amino acid hydrolysate, obtained in the acid hydrolysis of the distillery dreg proteins, in the amount 70 ml/l (360 mg/l of amino nitrogen). Starter yeast biomass S. cerevisiae was introduced to the substrate in the amount of 5 g/l. The fermentation was carried out at 38°C and a pH of 6.0. The speed of fermentation was 3.5 1/kg*h, concentration of ethanol at the end of fermentation was 8.8% by vol., isopentanols concentration 240 mg/l, and isobutanol concentration 140 mg/l. Total content of C₃-C₅ alcohols was 0.65% of the volume of ethanol.

Ethanol, C₃-C₅ alcohols and other volatile components were distilled off from the after-fermentation culture liquid (mash).

Suspended substances of the distillery dreg, after extractions of alcohol, obtained in the fermentation of carbohydrates yielded in starch hydrolysis, were condensed to a dry substance content of 5-10%. Sulphuric acid was added in an amount providing 0.2% concentration of H₂SO₄. After that acid hydrolysis of proteins of the distillery dreg after extraction of alcohol was carried out at 90°C. The amino acid hydrolysate thus obtained having a concentration of amino nitrogen of 2000-6000 mg/l was used as nitrogen nutrition of the yeast in fermentation of carbohydrate substrates.

A mixture of butyl alcohols was separated from C₂-C₅ alcohols and other volatile components and dehydrated in the presence of an Al₂O₃ catalyst at 250±50°C. Isobutylene obtained in the dehydration was mixed with synthesis gas obtained from biogas and having a ratio CO:H₂=1:1 and directed to the reactor with cobalt catalyst. Temperature in the reactor was kept at 160±20°C and pressure at 30±10 MPa. The mixture of amyl aldehydes thus obtained was directed to the reactor with Ni catalyst and hydrogenated at 150±50°C and a pressure 1-2 MPa by hydrogen produced from biomass, to obtain a mixture of amyl alcohols.

Furthermore, amyl aldehydes can be first condensed into isodecene aldehydes, which are then hydrogenated into isodecanols in the presence of Ni catalyst using hydrogen produced from biomass.

### EXAMPLE 17

Crushed wheat grain was mixed with water in ratio of 1:3.5. Enzymatic hydrolysis of the grain starch was accomplished using in the first stage thermostable amylase Zy-majunt-340C (pH 6.5, 90°C, consumption 0.25 ml per 1 kg of the grain starch), and in the second stage glycoamylase Glucozym L-400C (pH 5.0, 60°C, consumption 0.8 ml per 1 kg of the grain starch). Industrial enzymes produced by Ende Industries Inc., USA have been used. As a result of the enzymatic hydrolysis the concentration of carbohydrates in the substrate was 16%. To the substrate were added superphosphate in the amount providing a content of P₂O₅ of 200 mg/l, and aminoacid hydrolysate, obtained in the enzymatic hydrolysis of protein of the distillery dreg purified of ammonia and asparagines by known methods of ion exchange, in the amount 100 ml/l (400 mg/l of amino nitrogen). Starter yeast biomass S. cerevisiae was introduced to the substrate at a concentration of 5 g/l. The fermentation was carried out at a temperature of 38°C and a pH of 6.0. The speed of fermentation was 3.6 1/kg*h, the concentration of ethanol at the end of fermentation was 8.7% by vol., isopentanols concentration 920 mg/l, and isobutanol concentration 480 mg/l. The total content of C₃-C₅ alcohols reached 2.3% of the ethanol volume.

Ethanol, C₃-C₅ alcohols and other volatile components were distilled off from the after-fermentation culture liquid (mash).

Suspended substances of the distillery dreg, after extraction of alcohol, obtained in the fermentation of carbohydrates obtained in starch hydrolysis, were condensed to a dry substance content of 5-10%. After that enzymatic hydrolysis of the distillery dreg proteins was carried out using at the first stage endopeptidase Papain 30000USP-U/g, EC 3.4.22.2 (pH=5.5, 60°C; consumption 0.1 g per 1 kg of dry substance of the distillery dreg after extraction of alcohol) and at the second stage exopeptidase Carboxypeptidase A EC 3.4.17.1 (pH=7.5, 30°C, consumption 0.25 g per 1 kg of dry substance of the distillery dreg after extraction of alcohol) was carried out; the amino acid hydrolysate thus obtained having a concentration of amino nitrogen 2000-6000 mg/l was treated by ion exchange to extract ammonia nitrogen and asparagines; after that the mixture of amino acids free of asparagines and ammonia nitrogen was used as nitrogen nutrition of the yeast in fermentation of carbohydrate substrates.

The mixture of amyl alcohols was separated from C₂-C₅ alcohols and other volatile components and dehydrated at a temperature of 250±50°C in the presence of Al₂O₃ catalyst. The mixture of pentenes obtained in dehydration was mixed with synthesis gas obtained from biogas and having a ratio of CO:H₂=1:1 and directed to the reactor with cobalt-rhodium catalyst. The temperature in the reactor was kept at 90±10°C and the pressure at 2±1 MPa. Hexyl aldehydes thus obtained in the reactor were directed to the reactor with Ni catalyst and hydrogenated at 150±50°C and a pressure 1-2 MPa, using hydrogen produced from biomass, to obtain a mixture of hexyl alcohols.

Besides that, hexyl aldehydes can be first condensed into isododecene aldehydes, with the subsequent hydrogenation into isododecanols in the presence of Ni catalyst using hydrogen produced from biomass.

### EXAMPLE 18

Sugar cane molasses with a saccharose concentration of 46% was diluted with water to a saccharose concentration of 18%, acidified by sulphuric acid to pH 5.5, with subsequent addition of aminoacid hydrolysate, obtained in acid hydrolysis of protein of the distillery dreg free of alcohol, purified of ammonium and asparagines by the known methods of ion exchange, in the amount 90 ml/l (370 mg/l of amino nitrogen) and yeast starter biomass S. cerevisiae in the amount of 5 g/l. The fermentation was carried out at 38°C and pH 5.5. The speed of fermentation was 4.0 1/kg*h, C₂-C₅ alcohols concentration at the end of fermentation was 8.95% vol., including 0.2% vol. of C₃-C₅ alcohols, which amounts to 2.2% of the volume of ethanol.

C₂-C₅ alcohols were distilled off from the after-fermentation culture liquid (mash).

Suspended substances of the distillery dreg free of alcohol, obtained in the fermentation of carbohydrates obtained in starch hydrolysis, were condensed to a dry substance content of 5-10%.Hydrochloric acid was added in an amount providing a HCl concentration of 0.5%. Acid hydrolysis of protein of the distillery dreg, after extraction of alcohol, was carried out at 40°C; the amino acid hydrolysate thus obtained having a concentration of amino nitrogen 2000-6000 mg/l was treated by ion exchange to extract ammonia nitrogen and asparagine; after that the mixture of amino acids free of asparagine and ammonia nitrogen was used as nitrogen nutrition of the yeast in fermentation of carbohydrate substrates.

The mixture of C₂-C₅ alcohols obtained in the fermentation of molasses is dehydrated in the presence of Al₂O₃ catalyst at 300±100°C.

The mixture of unsaturated C₂-C₅ hydrocarbons obtained in dehydration was mixed with synthesis gas obtained from biogas and having a ratio CO:H₂=1:1 and directed to the reactor with cobalt-rhodium catalyst. The temperature in the reactor was kept at 90±10°C and pressure at 2±1 MPa. A mixture of C₃-C₆ aldehydes was obtained in the reaction of hydroformylation. Propionic aldehyde was then extracted from the mixture of C₃-C₆ aldehydes and hydrogenated at 150±50°C and a pressure 1-2 MPa into n-propanol in the presence of Ni catalyst by the hydrogen of the renewable origin. Propanol is returned to the stage of C₂-C₅ alcohols dehydration. C₄-C₆ aldehydes mixture was first condensed to the mixture of unsaturated C₈-C₁₂ aldehydes, which was then hydrogenated in the presence of Ni catalyst into the mixture of saturated C₈-C₁₂ alcohols by the hydrogen obtained from the renewable raw material. C₈ alcohols are extracted from the mixture of C₈-C₁₂ alcohols and dehydrated at 250±50°C in the presence of Al₂O₃ into isooctane, which is followed by hydrogenation into a mixture of isooctane by the hydrogen obtained from the renewable raw material.

Besides that, the total of C₃-C₆ aldehydes mixture, obtained in the reaction of hydroformylation, can be first condensed into a mixture of unsaturated C₆-C₁₂ aldehydes, which are then hydrogenated with Ni catalyst by the hydrogen of the renewable origin into the mixture of saturated C₆-C₁₂ alcohols of iso-structure.

Saturated C₆-C₁₂ alcohols can then be dehydrated with Al₂O₃ at a temperature of 250±50°C into a mixture of unsaturated C₆-C₁₂ hydrocarbons. The unsaturated C₆-C₁₂ hydrocarbons obtained in dehydration with Ni catalyst by H₂ of renewable origin are hydrogenated into a mixture of saturated C₆-C₁₂ hydrocarbons of iso-structure.

Moreover, in the presence of a catalyst (metal halogenides) at 20-100°C or at 200±50°C without catalyst saturated C₆-C₁₂ hydrocarbons can be condensed with C₁-C₃ aldehydes into a mixture of unsaturated C₇-C₁₅ alcohols, which alcohols are then hydrogenated in the presence of Ni catalyst by the renewable hydrogen into a mixture of saturated C₆-C₁₂ alcohols of iso-structure.

### EXAMPLE 19

Beet molasses with a saccharose concentration of 46% was diluted with water to a saccharose concentration of 18%, acidified by sulphuric acid to pH 5.5, and then the alcohol yeast autolysate was added in an amount of 50 ml/l (350 mg/l of amino nitrogen) and the yeast starter biomass S. cerevisiae in the amount of 5 g/l. The fermentation was carried out at 38°C and pH 5.5. The speed of fermentation was 4.0 1/kg*h, C₂-C₅ alcohols concentration at the end of fermentation was 8.85% vol., including 0.1% of C₃-C₅ alcohols, that is 1.1% of the volume of ethanol.

C₂-C₅ alcohols were distilled from the after-fermentation culture liquid (mash). C₂-C₅ alcohols mixture obtained in the molasses fermentation was dehydrated with Al₂O₃ catalyst at 300±100°C.

The mixture of unsaturated C₂-C₆ hydrocarbons obtained in dehydration was mixed with synthesis gas obtained from biogas and having a ratio CO:H₂=1:1 and directed to the reactor with cobalt-rhodium catalyst. The temperature in the reactor was kept at 90±10°C and the pressure at 2±1 MPa. In the reaction of hydroformylation a mixture of C₃-C₆ aldehydes was obtained. Propionic aldehyde is extracted from the mixture of C₃-C₆ aldehydes and hydrogenated at 150±50°C and a pressure 1-2 MPa into propanol in the presence of Ni catalyst by H₂ of renewable origin. Propanol is returned to the stage of C₂-C₅ alcohols dehydration. N-butyl aldehyde is extracted from said aldehydes mixture and condensed to 2-ethyl hexynal, which is then hydrogenated with Ni catalyst by the hydrogen of renewable origin into 2-ethylhexanol.

Besides that, C₅ aldehydes of iso-structure can be extracted from the mixture of C₄-C₆ aldehydes and converted into the corresponding amylenes, which in interaction with methanol form isoamylmethyl esters. The remaining mixture of C₄-C₆ aldehydes is condensed into unsaturated C₈-C₁₂ aldehydes of iso-structure, which are then hydrogenated in the presence of Ni catalyst by the hydrogen of renewable origin into a mixture of C₈-C₁₂ alcohols. Thus obtained C₈-C₁₂ alcohols can be dehydrated with Al₂O₃ and then hydrogenated in the presence of Ni catalyst at 250±50°C by hydrogen of renewable origin into the corresponding saturated C₈-C₁₂ hydrocarbons of iso-structure.

### EXAMPLE 20

Crushed wheat grain was mixed in a ratio of 1:10 by weight with water heated up to 80°C and kept at this temperature for 10 minutes, after which the temperature was elevated to 100°C and the mixture was let to stand for another 30 minutes. The substrate thus prepared was directed for sterilization in autoclave at 150°C during 60 minutes, after which the substrate is cooled down to 37°C. As a result of the overcooking of the flour the starch concentration in the substrate reached about 6%. To the substrate were added: the amino acid leucine in an amount of 750 mg/l and amino acid valine in the amount of 560 mg/l (amino nitrogen content of 150 mg/l). Starter bacteria biomass Clostridium acetobutylicum was introduced to the substrate at a concentration of 5 g/l. The fermentation was carried out at 37°C and a pH=5.5. The speed of fermentation was 3.5 1/kg*h, concentration of alcohols at the end of fermentation was 1.85% by volume, including 0.22% vol. of ethanol, 0.01% vol. of isopropanol, 0.03% vol. of isobutanol, 1.54% vol. of n-butanol, 0.05% vol. of isopenthanol, and concentration of acetone at the end of fermentation was 0.9% vol.

The mixture of C₂-C₅ alcohols and acetone obtained in the starch fermentation was, after separation of acetone, dehydrated with Al₂O₃ catalyst at 300±100°C. The mixture of unsaturated C₂-C₅ hydrocarbons obtained in dehydration was mixed with synthesis gas, originating from biogas and having a ratio of CO:H₂=1:1, and directed to the reactor with cobalt-rhodium catalyst. The temperature in the reactor was kept at 90±10°C and the pressure at 2±1 MPa.

A mixture of C₃-C₆ aldehydes is obtained in the reaction of hydroformylation. The mixture of C₃-C₆ aldehydes with added acetone is hydrogenated at 150±50°C and a pressure 5±1 MPa with Ni-catalyst by the hydrogen obtained in fermentation into the mixture of corresponding C₃-C₆ alcohols. C₃-C₄ alcohols are extracted from the said mixture and returned to the dehydration stage.

The remaining C₅-C₆ alcohols, having iso-structure, are dehydrated into the corresponding unsaturated hydrocarbons and, after interaction with methanol, are converted into isoamylmethyl and isoamylcaprylmethyl ethers. Wherein methanol used in the process is obtained from the carbon dioxide obtained at the stage OF fermentation and biogas obtained in processing of the fermentation waste.

The remaining C₅-C₆ alcohols of normal structure were dehydrated into the corresponding C₁₀-C₁₂ ethers.

### EXAMPLE 21

Crushed corn grain was mixed with water warmed up to 80°C in ratio of 1:10 by weight and kept at this temperature for 10 minutes, after which the temperature was elevated to 100°C and the mixture was let to stand for 30 minutes. The substrate thus prepared is directed for sterilization in autoclave at 150°C for 60 minutes, after which the substrate is cooled down to 38°C. As a result of the overcooking of the flour the starch concentration in the substrate reached about 6%. To the substrate were added acid hydrolysate of the yeast, after extraction of ammonia by known methods of ion exchange, in the amount 120 ml/l (360 mg/l of amino nitrogen). Starter bacteria biomass Clostridium butylicum and Clostridium acetobutylicum (1:4) was introduced to the substrate at a concentration of 5 g/l. The fermentation was carried out at 37°C and a pH=5.5. The speed of fermentation was 4.0 1/kg*h, concentration of alcohols at the end of fermentation was 2.0% by volume, including 0.22% by volume of ethanol, 0.15% by volume of isopropanol, 0.02% by volume of isobutanol, 1.58% by volume of n-butanol, 0.03% by volume of isopenthanol, and concentration of acetone at the end of fermentation was 0.95% by volume.

The mixture of C₂-C₅ alcohols and acetone obtained in the starch fermentation is, after separation of acetone, dehydrated with Al₂O₃ catalyst at 300±100°C. The mixture of unsaturated C₂-C₅ hydrocarbons obtained in dehydration is mixed with synthesis gas, originating from biogas and having a ratio of CO:H₂=1:1, and directed to the reactor with cobalt catalyst modified by the phosphorus compounds. The temperature in the reactor is kept at 175±25°C and a pressure 7.5±2.5 MPa.

In the reaction of hydroformylation a mixture of C₃-C₆ aldehydes is obtained. C₄ and C₅ aldehydes of normal structure are separated from said mixture. The remaining C₃-C₆ aldehydes with added acetone are hydrogenated at 150±50°C and a pressure of 5±1 MPa in the presence of Ni-catalyst by the hydrogen obtained in the fermentation into the mixture of the corresponding C₃-C₆ alcohols. C₅-C₆ alcohols are extracted from said mixture; these are alcohols of iso structure.

These alcohols are dehydrated into the corresponding unsaturated hydrocarbons and, after interaction with methanol, are converted into isoamylmethyl and isoamylcaprylmethyl ethers. The methanol used in the process is obtained from the carbon dioxide obtained at the stage of fermentation and biogas is obtained in processing of the fermentation waste.

C₄-C₅ aldehydes of normal structure are condensed into unsaturated C₈-C₁₀ aldehydes, which are then hydrogenated at 150±50°C and a pressure of 1-2 MPa in the presence of Ni catalyst by hydrogen obtained in the fermentation into saturated C₈-C₁₀ alcohols.

### EXAMPLE 22

Crushed wheat grain was mixed with water in ratio of 1:3.5. Enzymatic hydrolysis of the grain starch was accomplished using in the first stage thermostable amylase Zy-majunt-340C (pH 6.5, 90°C, consumption 0.25 ml per 1 kg of the grain starch), and in the second stage glycoamylase Glucozym L-400C (pH 5.0, 60°C, consumption 0.8 ml per 1 kg of the grain starch). Industrial enzymes produced by Ende Industries Inc., USA have been used. As a result of the enzymatic hydrolysis the concentration of carbohydrates in the substrate was 16%. Superphosphate was added to the substrate in an amount providing a content of P₂O₅ of 200 mg/l, the amino acid leucine in an amount of 2000 mg/l and amino acid valine in an amount of 1500 mg/l (amino nitrogen content of 390 mg/l). Starter yeast biomass S. cerevisiae was introduced to the substrate at a concentration of 5 g/l. The fermentation was carried out at 38°C and a pH of 6.0. The speed of fermentation was 3.5 1/kg*h, the concentration of ethanol at the end of fermentation was 8.8% by vol., isopentanol concentration 1250 mg/l, and isobutanol concentration 910 mg/l, the total content of C₄-C₅ alcohols was 2.95% of the volume of ethanol.

C₂-C₅ alcohols were distilled from the after-fermentation culture liquid (mash). The mixture of C₂-C₅ alcohols, obtained in the starch fermentation, was oxidized in the presence of silver catalyst at a temperature of 450-550°C by a mixture of oxygen and carbon dioxide, obtained in the biosynthesis of C₂-C₅ alcohols, to obtain a mixture of C₂-C₅ aldehydes. The C₂-C₅ aldehydes obtained in the oxidation were condensed into unsaturated C₄-C₁₅ aldehydes, which were hydrogenated in the presence of copper catalyst into a mixture of saturated C₄-C₁₅ aldehydes. The C₄-C₅ aldehydes were extracted from said mixture and returned to the condensation stage, and C₆-C₁₅ aldehydes were used for the extraction of individual aldehydes or hydrogenated in the presence of Ni-catalyst into a mixture of saturated C₆-C₁₅ alcohols. The latter can by means of dehydration and hydrogenation be converted into the mixture of saturated hydrocarbons. Besides that, both saturated and unsaturated C₆-C₁₅ aldehydes can by oxidized into the corresponding acids. For oxidation of aldehydes into fatty acids we used carbon dioxide from the process of biosynthesis. Oxidation of aldehydes into fatty acids was carried out in the presence of manganese acetate catalyst in the liquid phase and at 50-150°C and a pressure 0.05 MPa or in a gas phase at 150-250°C and a pressure 0.5 MPa. In contrast to the known methods, to the oxidation has been supplied heated to 50-150°C steam-gas mixture of aldehydes and carbon dioxide. Utilisation of said mixture gives a possibility to use for the oxidation oxygen or a mixture of oxygen with carbon dioxide.

### EXAMPLE 23

Chopped spruce wood (cellulose-containing plant material) was treated by acid hydrolysis at 180°C, a sulphuric acid concentration of 0.5%, a ratio of water to wood of 12:1, during 1.5 hours. The wood hydrolysate was neutralized with lime to a pH=4.5, and separated from lignin and gypsum residues. To the carbohydrate substrate thus obtained, having a hexose sugar concentration of 3.2% and pentose sugar concentration of 0.8%, superphosphate was added in an amount of P₂O₅ of 120 mg/l, the yeast autolysate, previously purified of ammonia and asparagines by known methods of ion exchange, in an amount of 45 ml/l of substrate (135 mg/l of amino nitrogen), and 5 g/l of the starter yeast biomass S. cerevisiae. The fermentation was carried out at 38°C and pH=6. Fermentation speed was 4.0 1/kg*h, ethanol concentration at the end of the fermentation was 1.5% vol., concentration of isopentanols was 210 mg/l, concentration of isobutanol was 130 mg/l, and total content of C₃-C₅ alcohols was 2.95% of the volume of ethanol.

C₂-C₅ alcohols were distilled off from the after-fermentation culture liquid (mash). The mixture of C₂-C₅ alcohols obtained in the fermentation of hexose sugars was oxidized at 450-550°C in the presence of a silver catalyst by the mixture of oxygen and carbon dioxide, obtained in the biosynthesis of C₂-C₅ alcohols, to obtain a mixture of C₂-C₅ aldehydes. The C₂-C₅ aldehydes obtained in oxidation are condensed in the presence of a 0.5% solution of sodium hydroxide at 0°C with furfural. The obtained mixture of unsaturated aldehydes is then hydrogenated in the presence of copper chrome catalyst at 100±50°C and a pressure of 0.1-5 MPa into a mixture of furyl-containing alcohols. The latter are subsequently hydrogenated at a temperature of 100±50° and a pressure of 5-10 MPa in the presence of nickel catalyst into a mixture of saturated alcohols containing tetrahydrofurane cycles.

### EXAMPLE 24

Chopped potatoes were mixed with water in a ratio of 1:1. Enzymatic hydrolysis of the potatoe starch was accomplished using in the first stage thermostable amylase Zy-majunt-340C (pH 6.5, 90°C, consumption 0.25 ml per 1 kg of the potatoes starch), and in the second stage glycoamylase Glucozym L-400C (pH 5.0, 60°C, consumption 0.8 ml per 1 kg of the potatoes starch). Industrial enzymes produced by Ende Industries Inc., USA have been used. As a result of the enzymatic hydrolysis the concentration of carbohydrates in the substrate reached 8.0%. To the substrate were added: superphosphate in the amount providing a content of P₂O₅=200 mg/l, the amino acid leucine in the amount of 1000 mg/l, and amino acid valine in the amount of 750 mg/l (amino nitrogen content of 195 mg/l). Starter yeast biomass S. cerevisiae was introduced to the substrate in the amount of 5 g/l. The fermentation was carried out at 38°C and pH=6.0. The speed of fermentation was 4.0 1/kg*h, ethanol concentration at the end of fermentation was 4.3% by vol., isopenthanols concentration 630 mg/l, and isobutanol concentration 460 mg/l. Total content of C₄-C₅ alcohols was 3.0% of the volume of ethanol.

C₂-C₅ alcohols were distilled off from the after-fermentation culture liquid (mash). The mixture of C₂-C₅ alcohols obtained in the fermentation was dehydrated at 300±100°C in the presence of Al₂O₃ catalyst. The mixture of unsaturated C₂-C₅ hydrocarbons obtained in the dehydration is mixed with synthesis gas, originating from biogas and having a ratio CO:H₂=1:1, and then directed to the reactor containing cobalt catalyst modified by phosphorous compounds. Temperature in the reactor is 175±25°C and a pressure of 7.5±2.5 MPa. As a result of the reaction of hydroformylation a mixture of C₃-C₆ aldehydes is obtained. The mixture of C₃-C₆ aldehydes is hydrogenated in the presence of Ni catalyst by the hydrogen produced from the renewable raw material into the mixture of C₃-C₆ alcohols, which are then returned back to the stage of dehydration.

The process is repeated until C₈ aldehydes appear in the mixture of aldehydes. After C₈ aldehydes appear in the mixture of aldehydes the process can be carried out in two routes. In the first route C₈ aldehydes are extracted and condensed into unsaturated C₁₆ aldehydes and then hydrogenated to saturated C₁₆ alcohols, which, if it is needed, are further processed into saturated C₁₆ hydrocarbons. In the second route the extracted C₈ aldehydes can be directly hydrogenated in the presence of Ni catalyst by renewable hydrogen into a mixture of C₈ alcohols, which are then converted into the mixture of C₈ hydrocarbons.

### EXAMPLE 25

Chopped potatoes were mixed with water in a ratio of 1:1. Enzymatic hydrolysis of the potatoe starch was accomplished using in the first stage thermostable amylase Zy-majunt-340C (pH=6.5, 90°C, consumption 0.25 ml per 1 kg of the potatoes starch), and in the second stage glycoamylase Glucozym L-400C (pH=5.0, 60°C, consumption 0.8 ml per 1 kg of the grain starch). Industrial enzymes produced by Ende Industries Inc., USA have been used. As a result of the enzymatic hydrolysis the concentration of carbohydrates in the substrate reached 8.0%. To the substrate were added: superphosphate in an amount providing a content of P₂O₅=200 mg/l, and acid hydrolysate of the yeast, treated by ion exchange for removal of asparagine and ammonium salts, in the amount 130 ml/l (390 mg/l of amino nitrogen). Starter yeast biomass S. cerevisiae was introduced to the substrate in the amount of 5 g/l. The fermentation was carried out at 38°C and a pH=6.0. The speed of fermentation was 4.0 1/kg*h, ethanol concentration at the end of fermentation was 4.4% by vol., isopenthanol concentration 560 mg/l, and isobutanol concentration 340 mg/l. The total content of C₃-C₅ alcohols reached 2.65% of the volume of ethanol.

C₂-C₅ alcohols were distilled off from the after-fermentation culture liquid (mash). The mixture of C₂-C₅ alcohols obtained in the fermentation was dehydrated at 300±100°C in the presence of Al₂O₃ catalyst. The mixture of unsaturated C₂-C₅ hydrocarbons obtained in the dehydration was mixed with synthesis gas, originating from biogas and having a ratio CO:H₂=1:1, and directed to the reactor containing cobalt catalyst modified by phosphorous compounds. The temperature in the reactor was 175±25°C and the pressure 7.5±2.5 MPa. As a result of the reaction of hydroformylation a mixture of C₃-C₆ aldehydes was obtained. The mixture of C₃-C₆ aldehydes was hydrogenated in the presence of Ni catalyst by renewable hydrogen into a mixture of C₃-C₆ alcohols, which were then returned back to the stage of dehydration.

The process was repeated until C₈ aldehydes appear in the mixture of aldehydes. After that C₈ aldehydes were condensed into unsaturated C₁₆ aldehydes and then hydrogenated into saturated C₁₆ alcohols. The latter were oxidized at 200-300°C in the presence of silver catalyst by the mixture of oxygen and carbon dioxide, obtained in biosynthesis of C₂-C₅ alcohols, to obtain a mixture of unsaturated C₁₆ acids. The obtained mixture of unsaturated C₁₆ acids was etherified by methanol in the presence of acid catalyst into a mixture of methyl esters of unsaturated C₁₆ acids. The methyl esters of unsaturated C₁₆ acids were subsequently hydrogenated at a temperature of 125-200°C by renewable hydrogen in the presence of Ni or Cu catalyst into a mixture of methyl esters of saturated C₁₆ acids.

### EXAMPLE 26

Beet molasses with a saccharose concentration of 46% was diluted by water to a saccharose concentration of 18% and acidified by sulphuric acid to pH=5.5.Then, the following were added: the acid yeast hydrolysate, which had been freed from asparagine and ammonium salts by ion exchange, in the amount 120 ml/l (360 mg/l of amino nitrogen), and yeast starter biomass S. cerevisiae in the amount of 5 g/l. The fermentation was carried out at 38°C and pH=5.5. The speed of fermentation was 3.6 1/kg*h, the concentration of ethanol at the end of fermentation was 8.7% vol., concentration of isopentanols 1000 mg/l, isobutanol 490 mg/l, and total content of C₃-C₅ alcohols reached 2.2% of the volume of ethanol.

C₂-C₅ alcohols were distilled off from the after-fermentation culture liquid (mash). The C₂-C₅ alcohols mixture obtained in the molasses fermentation was dehydrated with Al₂O₃ catalyst at 300±100°C.

The mixture of unsaturated C₂-C₅ hydrocarbons obtained on dehydration was mixed with synthesis gas obtained from biogas and having a ratio CO:H₂=1:1 and directed to the reactor with cobalt-rhodium catalyst. The temperature in the reactor was kept at 90±10°C and the pressure at 2±1 MPa. As a result of the reaction of hydroformylation a mixture of C₃-C₆ aldehydes was obtained. Propionic aldehyde was extracted from the mixture of C₃-C₆ aldehydes and hydrogenated into propanol in the presence of Ni catalyst by hydrogen of renewable origin. Propanol is then returned to the stage of C₂-C₅ alcohols dehydration. The mixture of C₄-C₆ aldehydes is first condensed into a mixture of unsaturated C₈-C₁₂ aldehydes, which is then hydrogenated by renewable hydrogen in the presence of Ni catalyst into a mixture of saturated C₈-C₁₂ alcohols. C₈ alcohols are extracted from the mixture of C₈-C₁₂ alcohols and then dehydrated in the presence of Al₂O₃ at a temperature of 200±25° into isooctanes, which are then hydrogenated by renewable hydrogen in the presence of Ni catalyst into a mixture of isooctanes.

Moreover, the whole mixture of C₃-C₆ aldehydes obtained in the reaction of hydroformylation is first condensed into a mixture of unsaturated C₆-C₁₂ aldehydes, which are then hydrogenated by renewable hydrogen in the presence of Ni catalyst into a mixture of saturated C₆-C₁₂ alcohols of iso-structure. Saturated C₆-C₁₂ alcohols are then dehydrated in the presence of Al₂O₃ at 250±50°C into a mixture of unsaturated C₆-C₁₂ hydrocarbons. Unsaturated C₆-C₁₂ hydrocarbons, obtained in dehydration, are mixed with methanol, obtained from carbon dioxide obtained in biosynthesis of C₂-C₅ alcohols, and processed at 200±100°C and a pressure of 0.1±10 kPa by carbon oxide, obtained from carbon dioxide obtained in biosynthesis of C₂-C₅ alcohols, in the presence of ferrous, nickel, cobalt, or rhodium carbonyls promoted by halogen derivatives. Thus, C₆-C₁₂ methyl esters of saturated acids are obtained.

### EXAMPLE 27

Crushed wheat grain was mixed with water in a ratio of 1:3.5. Enzymatic hydrolysis of the grain starch was carried out using in the first stage thermostable amylase Zy-majunt-340C (pH=6.5, 90°C, consumption 0.25 ml per 1 kg of the grain starch) and in the second stage glucoamylase Glucozym L-400C (pH=5.0; 60°C; consumption 0.8 ml per 1 kg of the grain starch). Industrial enzymes produced by Ende Industries Inc., USA have been used. As a result of the enzymatic hydrolysis, the concentration of carbohydrates in the substrate reached 16%. To the substrate were added sodium hydrogensulphite in an amount providing 3-4% content of NaHSO₃, superphosphate in an amount providing a P₂O₅ content of 200 mg/l, amino acid leucine in an amount of 2000 mg/l, and amino acid valine in the amount of 1500 mg/l (amino nitrogen content of 390 mg/l). The yeast starter biomass S. cerevisiae was introduced to the substrate in the amount of 5 g/l. The fermentation was carried out at 38°C and pH=6.0. The speed of fermentation was 3.5 1/kg*h, glycerine concentration at the end of fermentation was 3.0% vol., ethanol concentration 4.4% vol., acetaldehyde concentration 2.2% vol., isopenthanols concentration 0.15% vol., and isobutanol concentration 0.11% vol.

C₂-C₅ alcohols were distilled off from the after-fermentation culture liquid (mash). These alcohols can be processed into higher hydrocarbons as described in the foregoing examples. Glycerine and acetaldehyde were subsequently extracted from the after-fermentation culture liquid (mash) and acetalised at 0-50°C and a pressure 0.1-0.5MPa in the presence of hydrochloric acid or zinc chloride as a catalyst, to obtain 1,2-glycerineacetal acetaldehyde (2-methyl-4-oxymethyl-1,3-dioxane). 1,2-glycerineacetal acetaldehyde can be used as a component for motor fuels.

### EXAMPLE 28

Crushed wheat grain was mixed with water in a ratio of 1:3.5. Enzymatic hydrolysis of the grain starch was carried out using in the first stage thermostable amylase Zy-majunt-340C (pH=6.5; 90°C; consumption 0.25 ml per 1 kg of the grain starch) and in the second stage glucoamylase Glucozym L-400C (pH=5.0; 60°C; consumption 0.8 ml per 1 kg of the grain starch). Industrial enzymes produced by Ende Industries Inc., USA have been used. As a result of the enzymatic hydrolysis, the carbohydrates concentration in the substrate reached 16%. To the substrate were added sodium hydrogensulphite in an amount providing 3-4% content of NaHSO₃, superphosphate in an amount providing a P₂O₅ content of 200 mg/l, and aminoacid hydrolysate, obtained in the enzymatic hydrolysis of protein of the distillery dreg free of alcohol, which had previously been purified of ammonia and asparagines by known methods of ion exchange, in an amount of 100 ml/l (400 mg/l of amino nitrogen). The yeast starter biomass S. cerevisiae was introduced to the substrate in the amount of 5 g/l. The fermentation was carried out at 38°C and pH=6.0. The speed of fermentation was 3.5 1/kg*h, glycerine concentration at the end of fermentation was 3.1% vol., the ethanol concentration 4.5% vol., acetaldehyde concentration 2.4% vol., isopenthanols concentration 0.12% vol., and the isobutanol concentration 0.06% vol.

C₂-C₅ alcohols were distilled off and acetaldehyde was extracted from the after-fermentation culture liquid (mash). After that glycerine was extracted from the after-fermentation culture liquid (mash) and mixed with vegetable and/or animal fats. This mixture was hydrogenated in the presence of copper-chrome, zinc-chrome, nickel-chrome catalysts at 300±100°C and a pressure of 10-30 MPa into a mixture of n-propyl alcohol, higher C₆-C₂₀ alcohols and C₆ and higher hydrocarbons. The hydrogenation was carried our using hydrogen obtained from biomass and/or by biochemical method in the fermentation of carbohydrate substrates and/or from the water obtained in the processing of alcohols yielded in biosynthesis. Conversion of the water was carried out by the known methods. The mixture of glycerine and vegetable and/or animal fats can be also hydrogenated in the presence of catalysts containing precious metals, for example Pt, Pd, Re, Ru, Rh at 200±50°C and a pressure of 5-20 MPa.

### EXAMPLE 29

Crushed wheat grain was mixed with water in a ratio of 1:3.5. Enzymatic hydrolysis of the grain starch was carried out using in the first stage thermostable amylase Zy-majunt-340C (pH=6.5; 90°C; consumption 0.25 ml per 1 kg of the grain starch) and in the second stage glucoamylase Glucozym L-400C (pH=5.0; 60°C; consumption 0.8 ml per 1 kg of the grain starch). Industrial enzymes produced by Ende Industries Inc., USA have been used. As a result of the enzymatic hydrolysis, the carbohydrates concentration in the substrate reached 16%. To the substrate were added sodium hydrogensulphite in the amount providing 3-4% content of NaHSO₃, superphosphate in the amount providing P₂O₅ content of 200 mg/l, and aminoacid hydrolysate, obtained in the enzymatic hydrolysis of protein of the distillery dreg free of alcohol, and purified of ammonia and asparagines by known methods of ion exchange, in the amount of 90 ml/l (370 mg/l of amino nitrogen). The yeast starter biomass S. cerevisiae was introduced to the substrate in the amount of 5 g/l. The fermentation was carried out at 38°C and pH=6.0. The speed of fermentation was 3.5 1/kg*h, the glycerine concentration at the end of fermentation was 3.2% vol., ethanol concentration 4.3% vol., acetaldehyde concentration 2.4% vol., and concentration of C₃-C₅ alcohols 0.2% vol.

C₂-C₅ alcohols were distilled off and acetaldehyde was extracted from the after-fermentation culture liquid (mash). After that glycerine was extracted from the after-fermentation culture liquid (mash) and mixed with glycerine obtained in the saponification of fats; the mixture obtained was dehydrated in the presence of Al₂O₃ catalyst at 350±50°C. Acrolein obtained in dehydration of glycerine was directed to a reactor containing Ni catalyst and hydrogenated at 100±10°C and a pressure of 1-2 MPa into n-propyl alcohol using hydrogen obtained from biomass and/or obtained in the fermentation of carbohydrate substrates and/or obtained from the water, obtained in the processing of alcohols obtained in biosynthesis. Conversion of the water was carried out by known methods.

N-propyl alcohol thus obtained was brought together with C₂-C₅ alcohols obtained in biosynthesis; the mixture of lower C₂-C₅ alcohols thus obtained was condensed to obtain a mixture of C₄-C₁₅ alcohols, C₂-C₅ fatty acids, and C₄-C₁₀ esters. The process of condensation of the lower C₂-C₅ alcohols was carried out at 150±50°C and a pressure of 0.1-0.5 MPa in the presence of sodium alcoholates and Ni-Cr₂O₃ as a catalyst. Sodium alcoholates for this reaction were prepared from sodium hydroxide directly in the process of condensation. To increase the yield of the products of condensation the water obtained in the reaction was extracted in the form of a azeotrope mixture with non-condensed alcohols. C₂-C₅ fatty acids were separated from C₄-C₁₅ alcohols and C₄-C₁₀ esters and etherified in the presence of acid catalyst by the mixture of terpenes into a mixture of terpene esters of fatty C₂-C₅ acids. Non-condensed C₂-C₅ alcohols were dehydrated in the presence of Al₂O₃ catalyst at 300±50°C and mixed with terpenes, which terpenes previously had been heated to 200±50C in the presence of platinum. As a result of alkylation, which was carried out at a temperature of 0-10°C and a pressure of 0.5-1 MPa using as a catalyst 90-100% sulphuric acid, a mixture of C₁₂-C₁₅ hydrocarbons is obtained. The process of alkylation can also be carried out in the presence of AlCl₃ catalyst at 50-60°C and a pressure of 1-2 MPa. Terpene esters and higher hydrocarbons obtained from terpenes, lower C₂-C₅ alcohols and C₂-C₅ fatty acids were then used as components for motor fuels.

### EXAMPLE 30

Crushed wheat grain was mixed with water in the ratio 1:3.5. Enzymatic hydrolysis of the grain starch was carried out using in the first stage thermostable amylase Zyma-junt-340C (pH=6.5; 90°C; consumption 0.25 ml per 1 kg of the grain starch) and in the second stage glucoamylase Glucozym L-400C (pH=5.0; 60°C; consumption 0.8 ml per 1 kg of the grain starch). Industrial enzymes produced by Ende Industries Inc., USA have been used. As a result of the enzymatic hydrolysis, the carbohydrates concentration in the substrate reached 16%. To the substrate were added sodium hydroorthophosphate in an amount providing a 4% content of Na₂HPO₄, amino acid leucine in an amount of 2000 mg/l, and amino acid valine in an amount of 1500 mg/l. The yeast starter biomass S. cerevisiae was introduced to the substrate in the amount of 5 g/l. The fermentation was carried out at 38°C and pH=6.0. The speed of fermentation was 3.5 1/kg*h, the glycerine concentration at the end of fermentation was 4.5% vol., ethanol concentration 4.1% vol., acetic acid concentration 4.0% vol., isopenthanols concentration 0.15% vol., and isobutanol concentration 0.11% vol.

From the after-fermentation culture liquid (mash) C₂-C₅ alcohols were first distilled off and acetic acid extracted, which can be further processed into higher hydrocarbons as described in the foregoing examples. After that, glycerine was extracted from the after-fermentation culture liquid (mash) and dehydrated in the presence of Al₂O₃ catalyst at 350±50°C.

Acrolein obtained in the dehydration of glycerine was directed to the reactor containing CuO-Cr₂O₃ catalyst and hydrogenated at 175±25°C and a pressure of 1-5 MPa into a mixture of propionic aldehyde and n-propyl alcohol by hydrogen obtained from biomass and/or obtained from the water, obtained in dehydration of glycerine. Conversion of the water was carried out using known methods. Propionic aldehyde was extracted from the mixture obtained and can be further processed in two routes. The first route provides further condensing of propionic aldehyde into isohexene aldehyde with the subsequent hydrogenation in the presence of Ni catalyst at 150±10°C and a pressure of 1-5 MPa into isohexanol by hydrogen obtained from biomass and/or obtained from the water obtained in dehydration of glycerine. Conversion of the water is carried out by known methods.

The second possibility is to condense propionic aldehyde with C₂-C₅ alcohols, obtained in biosynthesis, into the corresponding propanals; or to condensate propionic aldehyde with n-propyl alcohol, obtained in hydrogenation of acroleine, wherein the ratio acrolein : propionic aldehyde by mole is 2:1, into dipropyl propanal. The latter is a good component for fuels for diesel and gas-turbine engines.

### EXAMPLE 31

Crushed wheat grain was mixed with water in the ratio 1:3.5. Enzymatic hydrolysis of the grain starch was carried out using in the first stage thermostable amylase Zy-majunt-340C (pH=6.5; 90°C; consumption 0.25 ml per 1 kg of the grain starch) and in the second stage glucoamylase Glucozym L-400C (pH=5.0; 60°C; consumption 0.8 ml per 1 kg of the grain starch). Industrial enzymes produced by Ende Industries Inc., USA have been used. As a result of the enzymatic hydrolysis, the carbohydrates concentration in the substrate reached 16%. To the substrate were added sodium hydroorthophosphate in an amount providing 4% content of Na₂HPO₄, and aminoacid hydrolysate, obtained in acid hydrolysis of protein of the distillery dreg free of alcohol, which had been purified of ammonia and asparagine by known methods of ion exchange, in the amount of 90 ml/l (370 mg/l of amino nitrogen). The yeast starter biomass S. cerevisiae was introduced to the substrate in the amount of 5 g/l. The fermentation was carried out at 38°C and pH=6.0. The speed of fermentation was 4.0 1/kg*h, the glycerine concentration at the end of fermentation was 4.7% vol., ethanol concentration 4.0% vol., acetic acid concentration 4.2% vol., and the concentration of C₃-C₅ alcohols 0.2%.

From the after-fermentation culture liquid (mash) C₂-C₅ alcohols were first distilled off and acetic acid was extracted, which can be further processed into higher hydrocarbons as described in the foregoing examples. After that, glycerine was extracted from the after-fermentation culture liquid (mash) and mixed with glycerine obtained in the saponification of fats. This mixture was dehydrated in the presence of Al₂O₃ catalyst at 350±50°C. Acroleine obtained in dehydration of glycerine was mixed with benzene and directed to the dimerization reactor, where, at 170±10°C and a pressure 1-2 MPa, in the presence of hydroquinone, the dimer of acroleine (2-formyl-3,4-dihydro-2H- pyran) was obtained. The dimer of acrolein (2-formyl-3,4-dihydro-2H-pyran) was separated from benzene and hydroquinone and hydrogenated in the presence of Ni catalyst at 150±10°C and a pressure of 5-10 MPa into tetrahydropyran-2-methanol by hydrogen obtained from biomass and/or by hydrogen obtained from the water obtained in dehydration of glycerine. Conversion of the water is carried out by known methods. Tetrahydropyran-2-methanol thus obtained is a good component for motor fuels for diesel and gas-turbine engines.

### EXAMPLE 32

Crushed wheat grain was mixed with water in the ratio 1:3.5. Enzymatic hydrolysis of the grain starch was carried out using in the first stage thermostable amylase Zy-majunt-340C (pH=6.5; 90°C; consumption 0.25 ml per 1 kg of the grain starch) and in the second stage glucoamylase Glucozym L-400C (pH=5.0; 60°C; consumption 0.8 ml per 1 kg of the grain starch). Industrial enzymes produced by Ende Industries Inc., USA have been used. As a result of the enzymatic hydrolysis, the carbohydrates concentration in the substrate reached 16%.

To the substrate were added: superphosphate in an amount providing a P₂O₅ content of 200 mg/l, and aminoacid hydrolysate, obtained in the enzymatic hydrolysis of protein of the distillery dreg free of alcohol, which had previously been purified from ammonia and asparagine by known methods of ion exchange, in the amount of 100 ml/l (400 mg/l of amino nitrogen). The yeast starter biomass S. cerevisiae was introduced to the substrate in the amount of 5 g/l. The fermentation was carried out at 38°C and pH=6.0. The speed of fermentation was 3.6 1/kg*h, ethanol concentration at the end of fermentation was 8.7% vol., isopentanols concentration 920 mg/l, isobutanol concentration 480 mg/l, and the total content of C₃-C₅ alcohols was 2.3% of the volume of ethanol.

Ethanol, C₃-C₅ alcohols and other volatile components were distilled off from the after-fermentation culture liquid (mash). C₃-C₅ alcohols and other volatile components can be further processed into higher hydrocarbons as described in the foregoing examples. Carbon dioxide obtained in the biosynthesis of alcohols was mixed with biogas, containing mainly methane, and with water steam and directed to the reactor for synthesis gas production. Conversion of the source mixture is carried out in the presence of NiO-Al₂O₃ catalyst at a temperature of 830-850°C. Thus, a gas mixture of the following composition is obtained: CO₂-4.8% vol; CO-24.7% vol.; H₂-68.0% vol.; CH₄-2.3% vol. Converted gas is then cooled down and compressed by the compressor to 5 MPa and directed to methanol synthesis. Methanol synthesis is carried out at 5 MPa and a temperature of 230-260°C in the presence of CuO-ZnO-Al₂O₃ (Cr₂O₃). Methanol obtained from carbon dioxide was mixed with ethanol obtained in biosynthesis and the thus obtained mixture was oxidized at 450-550°C in the presence of silver catalyst by the mixture of oxygen and carbon dioxide, obtained in the biosynthesis of C₂-C₅ alcohols, to obtain a mixture of acetaldehyde and formaldehyde. The mixture of acetaldehyde and formaldehyde was subsequently converted at 300-400°C in the presence of Al₂O₃ into acrolein. Acrolein was processed into higher hydrocarbons, including oxygen containing hydrocarbons, as described in the foregoing examples.

### EXAMPLE 33

Crushed wheat grain was mixed with water in the ratio 1:3.5. Enzymatic hydrolysis of the grain starch was carried out using in the first stage thermostable amylase Zy-majunt-340C (pH=6.5; 90°C; consumption 0.25 ml per 1 kg of the grain starch) and in the second stage glucoamylase Glucozym L-400C (pH=5.0; 60°C; consumption 0.8 ml per 1 kg of the grain starch). Industrial enzymes produced by Ende Industries Inc., USA have been used. As a result of the enzymatic hydrolysis, the carbohydrates concentration in the substrate reached 16%.

To the substrate were added: superphosphate in an amount providing a P₂O₅ content of 200 mg/l, and aminoacid hydrolysate, obtained in the enzymatic hydrolysis of protein of the distillery dreg free of alcohol, previously purified of ammonia and asparagine by known methods of ion exchange, in the amount of 100 ml/l (400 mg/l of amino nitrogen). The yeast starter biomass S. cerevisiae was introduced to the substrate in the amount of 5 g/l. The fermentation was carried out at 38°C and pH=6.0. The speed of fermentation was 3.6 1/kg*h, the ethanol concentration at the end of fermentation was 8.7% vol., isopentanols concentration 920 mg/l, isobutanol concentration 480 mg/l, and the total content of C₃-C₅ alcohols was 2.3% of the volume of ethanol.

Ethanol, C₃-C₅ alcohols and other volatile components were distilled off from the after-fermentation culture liquid (mash). Isobutyl and isoamyl alcohols were separated from the C₂-C₅ alcohols. The mixture of C₂-C₅ alcohols obtained after extraction of isobutyl and isoamyl alcohols was dehydrated in the presence of Al₂O₃ catalyst at 300±100°C, while isobutyl and isoamyl alcohols were dehydrated in the presence of Al₂O₃ catalyst at 250±50°C. Isobutene and isopentene thus obtained were then hydrogenated in the presence of Ni catalyst at 150±50°C and a pressure of 1-2 MPa into isobutene and isopentane by hydrogen obtained from biomass and/or by hydrogen obtained from the water obtained in dehydration of alcohols. Conversion of the water is carried out by the conventional methods. Isobutane and isopentane thus obtained were mixed with unsaturated C₂-C₅ hydrocarbons obtained in dehydration of the corresponding alcohols at 0-10°C and a pressure of 0.5-1 MPa in the reactor containing, as a catalyst, sulphuric acid. As a result of the synthesis a mixture of saturated C₆-C₁₀ hydrocarbons was obtained, which is a good component for gasoline fuels.

This alkylation process can also be carried out in the presence of AlCl₃ as a catalyst and a temperature of 50-60°C and a pressure of 1-2 MPa.

Moreover, unsaturated C₂-C₅ hydrocarbons obtained in dehydration of the corresponding C₂-C₅ alcohols were mixed with terpenes, which were in beforehand heated in the presence of platinum to 200±50°C. As a result of alkylation, which was carried out at 0-10°C and a pressure of 0.5-1 MPa using 90-100% sulphuric acid as a catalyst, a mixture of C₁₂-C₁₅ hydrocarbons was obtained. This process of alkylation can also be performed in the presence of AlCl₃ used as a catalyst at 50-60°C and a pressure 1-2 MPa. Higher C₁₂-C₁₅ hydrocarbons obtained from terpenes and lower C₂-C₅ alcohols were used as components of motor fuels.

### Example 34

Crushed corn grain was mixed with water heated up to 80°C in ratio 1:10 by weight and was let to stand at this temperature for 10 minutes, after which the temperature was elevated to 100°C and the mixture was let to stand for another 30 minutes. The substrate thus prepared is directed for sterilization in an autoclave at 150°C for 60 minutes, after which the substrate is cooled down to 37°C. As a result of the overcooking of the flour the starch concentration in the substrate reached about 6%. To the substrate was added aminoacid hydrolysate, obtained in the enzymatic hydrolysis of protein of the distillery dreg free of alcohol, which hydrolysate had previously been purified from ammonia by known methods of ion exchange, in the amount 100 ml/l (400 mg/l of amino nitrogen). After that starter bacteria biomass Clostridium butylicum and Clostridium acetobutylicum (in a ratio of 1:4) was introduced to the substrate in the concentration of 5 g/l. The fermentation was carried out at 37°C and a pH=5.5. The speed of fermentation was 4.0 l/kg*h, the concentration of alcohols at the end of fermentation was 2.05% vol., including 0.24% vol. of ethanol, 0.12% vol. of isopropanol, 0.03% vol. of isobutanol, 1.61% vol. of n-butanol, 0.05% vol. of isopenthanol, and concentration of acetone at the end of fermentation was 0.7% by volume.

The mixture of C₂-C₅ alcohols extracted from the culture liquid obtained in the fermentation of the grain starch can be processed into higher hydrocarbons as described in the foregoing examples. Acetone that has been distilled off from the culture liquid was treated by aldol and croton condensation to obtain a mixture of diacetone alcohol, mesityl oxide, phorone, and mesitylene. Mesityl oxide and isophorone were extracted from the mixture obtained and hydrogenated in the presence of Ni catalyst at 150±10°C and a pressure of 1-5 MPa into the corresponding C₆ and C₉ alcohols.

C₆ and C₉ alcohols, thus obtained, were brought together with diacetone alcohol and mesitylene and the obtained mixture was used as a component for gasoline.

Moreover, acetone distilled off from the culture liquid was condensed with glycerine, obtained in biosynthesis or in the saponification of fats, to produce acetone 1,2-glycerineketal (2,2-dimethyl-4-oxymethyl 1,3-dioxane). The latter was also used as a component for motor fuels.

## Claims

1. A method of producing hydrocarbons and oxygen-containing compounds from biomass, comprising the steps of: preparation of an aqueous carbohydrate substrate with a carbohydrate concentration of 3-20% comprising a source of nitrogen; fermenting the substrate using yeast or a bacterium selected from Clostridium acetobutylicum and Clostridium butylicum to an overall concentration of 1.5-10% of the following products C₁-C₅ alcohols, glycerine (glycerol), acetaldehyde, acetic acid and acetone; and separation of desired products from the fermentation medium, **characterized in that**, as a source of nitrogen, the amino acids leucine, isoleucine, or valine, or a mixture thereof is added to the aqueous carbohydrate substrate in an amount providing a content of amino nitrogen of from 120 to 420 mg/l of fermentation medium.

2. The method of claim 1, **characterized in that** the process of fermentation is carried out at a speed of 2.8-4.0 l/kg per hour.

3. The method of claim 1 or 2, **characterized in that** the carbohydrate substrate used is beet or cane molasses, saccharized starch of different kinds of grains or potatoes, wherein the starch is an acid starch hydrolysate, or enzymatic starch hydrolysate.

4. The method of any one of the previous claims, **characterized in that** the content of amino nitrogen in the medium is of from 320 to 400 mg/l.

5. The method of claim 4, **characterized in that** the content of amino nitrogen in the medium is of from 350 to 370 mg/l.

6. The method of any one of the previous claims, **characterized in** comprising the further steps of: condensing yeast obtained in the fermentation of carbohydrate substrate to a dry substance content of 5-10%; and autolysis of the yeast protein at 45-55°C for 24-48 hours for obtaining an autolysate exhibiting a content of amino nitrogen of 3000-8000 mg/l.

7. The method of any one of the previous claims, **characterized in** comprising the further steps of: condensing suspended substances contained in the fermentation medium after fermentation of carbohydrate substrate and separation of alcohol therefrom and to a dry substance content of 5-10%; and, either acid hydrolysis of the protein contained in said substances using sulphur or hydrochloric acids or enzymatic hydrolysis of the protein contained in said substances using proteolytic enzymatic preparations, for obtaining an acid hydrolysate of proteins exhibiting a content of amino nitrogen of 2000-6000 mg/l.

8. The method of any one of the previous claims, **characterized in** comprising the further steps of: aerobic cultivation of yeast using the water soluble substances contained in the fermentation medium after fermentation of carbohydrate substrate and separation of alcohol therefrom; condensing the yeast thus obtained to a dry substance content of 5-10%; and autolysis of the yeast protein at 45-55°C for 24-48 hours for obtaining an autolysate exhibiting a content of amino nitrogen of 3000-8000 mg/l.

9. The method of claim 1 or 2, **characterized in that** the carbohydrate substrate is an acid hydrolysate of cellulose-containing materials.

10. The method of claim 9, **characterized in** comprising the further steps of: aerobic cultivation of yeast with the pentose containing fermentation medium after fermentation of carbohydrate substrate and separation of alcohol therefrom; condensing the yeast thus obtained to a dry substance content of 5-10%; and autolysis of the yeast protein at 45-55°C for 24-48 hours for obtaining an autolysate of yeast protein exhibiting a content of amino nitrogen of 3000-8000 mg/l.

11. The method of any one of the previous claims, **characterized in that** a mixture of alcohols is separated from the fermentation medium by means of distillation, exhibiting an ethanol content of 96.9-99.35 a content of C₃-C₅ alcohols of 0.65-3.1% by volume.

12. The method of any one of the claims 1-10, **characterized in that** a product mixture is separated from the fermentation medium, exhibiting a content of acetone of 25.5-32.7, n-butanol of 56.0-58.5, ethanol of 7.3-8.7%, isopropanol of 0.4-4.4, isobutanol of 1.1-1.5, and isopentanol of 1.8-2.2% by volume.

13. The method of any one of the previous claims **characterized in** comprising the further step of: using C₁-C₅ alcohols, glycerin, acetaldehyde, and acetone obtained in biosynthesis in preparing a motor fuel.

14. The method of any one of the claims 5-8 and 10-13, **characterized in** comprising the further step of: drying up the excess autolysate of the yeast protein for use as an animal feed.

15. The method of any one of claims 5-8 and 10-14, **characterized in** comprising the further step of: biosynthesis of methane using suspended substances obtained in the acid or enzymatic hydrolysis or autolysis of protein with the excess hydrolysate as a substrate.

16. The method of any one of the previous claims, **characterized in** comprising the further step of: obtaining higher oxygen-containing compounds and/or non oxygen-containing hydrocarbons, including those having four and more carbon atoms in the molecule using the product mixture of C₁-C₅ alcohols, glycerin, acetaldehyde and acetone separated from fermentation medium.

17. The method of claim 16, **characterized in** comprising the further step of: using the compounds obtained in the method of claim 16 in preparation of motor fuels.

18. The method of claim 16 or 17, **characterized in** comprising the further steps of: dehydration of the product mixture of C₁-C₅ alcohols separated after fermentation in order to obtain unsaturated C₂-C₅ hydrocarbons; reacting said unsaturated C₂-C₅ hydrocarbons with synthesis gas in a hydroformylation reaction to obtain aldehydes; hydrogenation of said aldehydes into a mixture of higher alcohols, alternatively said aldehydes are first condensed into higher unsaturated aldehydes which are then hydrogenated into the corresponding higher saturated alcohols.

19. The method of claim 18 **characterized in** comprising the further steps of: preparing synthesis gas from biomass and/or from wastes obtained in the processing of the separated product mixture into higher hydrocarbons, C₂-C₆ acids and/or methane obtained by biochemical means or carbon dioxide obtained by biochemical means.

20. The method of any one of the claims 16-19, **characterized in** comprising the further steps of: oxidizing the product mixture of C₁-C₅ alcohols separated after fermentation in the presence of carbon dioxide, obtained by biochemical means, into a mixture of C₁-C₅ aldehydes; condensation of said aldehydes into a mixture of higher unsaturated aldehydes; and subsequent hydrogenation into a mixture of the corresponding higher saturated alcohols.

21. The method of any one of the claims 16-20, **characterized in** comprising the further steps of: dehydration of saturated C₄ and higher alcohols into the corresponding unsaturated hydrocarbons; and hydrogenation of said unsaturated hydrocarbons into the corresponding saturated C₄ and higher hydrocarbons.

22. The method of any one of the claims 16-21, **characterized in** comprising the further step of: dehydration of saturated C₃ and higher alcohols to obtain the corresponding ethers.

23. The method of any one of the claims 16-22, **characterized in** comprising the further step of: reacting unsaturated C₅-C₆ hydrocarbons of iso structure with methanol to obtain the corresponding methyl ethers.

24. The method of any one of the claims 16-23, **characterized in** comprising the further step of: oxidizing the product mixture of C₁-C₅ alcohols separated after fermentation in the presence of carbon dioxide, obtained by biochemical means, in order to obtain a mixture of aldehydes; condensation of said mixture into a mixture of higher unsaturated aldehydes; oxidizing said unsaturated aldehydes in the presence of carbon dioxide, obtained by biochemical means, into a mixture of higher unsaturated acids; and reacting said acids with methanol to obtain the corresponding methyl esters.

25. The method of claim 23, **characterized in** comprising the further steps of: hydrogenation of the higher unsaturated acids into higher saturated acids; and reacting said saturated acids with methanol to obtain the corresponding methyl esters.

26. The method of any one of the claims 23-25, **characterized in** comprising the further steps of: preparation of methanol using carbon dioxide obtained by biochemical means, methane obtained by biochemical means, and hydrogen obtained from biomass and/or by biochemical methods in fermentation of carbohydrate substrates, and/or from water obtained in processing of alcohols obtained in biosynthesis.

27. The method of claim 26, **characterized in** comprising the further step of: reaction of the methanol with fatty C₄ and higher acids to produce the corresponding esters.

28. The method of claim 27, **characterized in** comprising the further step of: oxidizing C₄-C₅ alcohols from the product mixture separated after fermentation to obtain C₄ and higher fatty acids; and/or biosynthesis of C₄-C₆ fatty acids; and/or extraction of fatty acids from tall oil; and/or saponification of fats in order to obtain fatty acids.

29. The method of any one of the claims 16-19, **characterized in** comprising the further steps of: dehydration of saturated C₄ and higher alcohols into the corresponding unsaturated C₄ and higher hydrocarbons; and reaction of said hydrocarbons with C₁ and higher fatty acids to obtain the corresponding esters.

30. The method of claim 28, **characterized in** comprising the further steps of: preparation of C₁ and higher fatty acids by oxidizing C₁-C₅ alcohols from the product mixture separated after fermentation; and/or preparation of C₂-C₆ fatty acids via biosynthesis; and/or extraction of fatty acids from tall oil; and/or preparation of fatty acids by saponification of fats.

31. The method of any one of the claims 18-20, **characterized in** comprising the further step of: reacting the unsaturated C₄ and higher hydrocarbons, obtained in the dehydration of the corresponding saturated alcohols, with C₂-C₅ alcohols obtained in biosynthesis to obtain the corresponding ethers.

32. The method of any one of the claims 16-20, **characterized in** comprising the further steps of: extraction of isobutane and isopentane from the mixture of saturated hydrocarbons; reaction with unsaturated C₂ and higher hydrocarbons obtained in the dehydration of the corresponding saturated alcohols to obtain saturated C₆ and higher hydrocarbons.

33. The method of claim 16 or 17, **characterized in** comprising the further steps of: processing vegetable and/or animal fats, and/or glycerin obtained in the saponification of fats, and/or glycerin obtained in biosynthesis into n-propyl alcohol; mixing said n-propyl alcohol with C₁-C₅ alcohols separated after fermentation; preparation of higher oxygen-containing compounds and/or non oxygen-containing hydrocarbons, including those having four and more carbon atoms in the molecule using said mixture.

34. The method of claim 16 or 17, **characterized in** comprising the further steps of: extraction of glycerin extracted from a product mixture of C₃-C₅ alcohols obtained in fermentation of carbohydrate substrates; dehydration of said glycerin into acrolein; hydrogenation of acrolein into propionic aldehyde and propyl alcohol; condensation of said propionic aldehyde with the C₃-C₅ alcohols obtained in fermentation of carbohydrate substrates, and propanol obtained in hydrogenation of acrolein, into the corresponding propanals; alternatively propionic aldehyde is first condensed into unsaturated isohexene aldehyde, which is then hydrogenated into the saturated alcohol isohexanol.

35. The method of claim 16 or 17, **characterized in** comprising the further steps of: extracting glycerin extracted from a product mixture of C₃-C₅ alcohols obtained in fermentation of carbohydrate substrates; dehydration of said glycerin into acrolein; condensation of acrolein into the acrolein dimer 2-formyl-3,4-dihydro-2H-pyran; hydrogenation of the acrolein dimer into tetrahydropyran-2-methanol; while using the remaining mixture of C₃-C₅ alcohols obtained in fermentation of carbohydrate substrates for obtaining higher oxygen-containing compounds and/or non oxygen-containing hydrocarbons, including those having in the molecule four and more carbon atoms.

36. The method of claim 16 or 17, **characterized in** comprising the further steps of: extraction of methanol and ethanol from the mixture of C₁-C₅ alcohols obtained in fermentation of carbohydrate substrates; adding methanol, produced from carbon dioxide obtained in fermentation of carbohydrate substrates, and hydrogen, derived from biomass; oxidation of said methanol and ethanol into formaldehyde and acetaldehyde, respectively; condensation of the obtained mixture of formaldehyde and acetaldehyde into acrolein; condensation of acrolein into acrolein dimer 2-formyl-4,4-dihydro-2H-pyran; hydrogenation of acrolein dimer into tetrahydro-pyran-2-methanol, while the remaining mixture of C₃-C₅ alcohols obtained in fermentation of carbohydrate substrates is used for obtaining higher oxygen-containing compounds and/or non oxygen-containing hydrocarbons, including those having four and more carbon atoms in the molecule.

37. The method of any one of the claims 16, 17, and 33, **characterized in** comprising the further steps of: condensation of the mixture of C₁-C₅ alcohols separated after fermentation, and/or n-propyl alcohol, obtained from glycerin, to produce saturated C₆ and higher alcohols, saturated C₅ and higher esters, and C₂ and higher fatty acids; while using any remaining lower alcohols, that did not condense, and gaseous products, obtained in the condensation, for producing higher oxygen-containing compounds and/or non oxygen-containing hydrocarbons, including those having in the molecule four and more carbon atoms.

38. The method of claim 37, **characterized in** comprising the further steps of: dehydration of the saturated C₆ and higher alcohols, obtained in condensation of C₁-C₅ alcohols, to obtain unsaturated C₆ and higher hydrocarbons; and hydrogenation of said unsaturated C₆ and higher hydrocarbons into saturated C₆ and higher hydrocarbons.

39. The method of claim 38, **characterized in** comprising the further steps of: reacting unsaturated C₆ and higher hydrocarbons, obtained in dehydration of the corresponding saturated alcohols, with non-condensed C₁-C₅ alcohols to obtain the corresponding C₇ and higher ethers.

40. The method of claim 39, **characterized in** comprising the further steps of: dehydration of non-condensed lower alcohols C₂-C₅ to obtain unsaturated C₂-C₅ hydrocarbons; alkylation of terpenes by unsaturated C₂-C₅ hydrocarbons to obtain C₁₂ and higher hydrocarbons.

41. The method of claim 37 or 38, **characterized in** comprising the further step of: reacting the C₂ and higher fatty acids, obtained in condensation of C₁-C₅ alcohols, with unsaturated C₆ and higher hydrocarbons, obtained in dehydration of the corresponding saturated alcohols, to obtain the corresponding C₈ and higher esters.

42. The method of claim 37, **characterized in** comprising the further step of: reacting the C₂ and higher fatty acids, obtained in the process of C₁-C₅ alcohols condensation, with terpenes to obtain the corresponding C₁₂ and higher esters.

43. The method of any one of the previous claims, **characterized in** comprising the further steps of: separation of acetone from the mixture of C₂-C₅ alcohols obtained in fermentation of carbohydrate substrates; treatment of the acetone by aldol and croton condensation to obtain a mixture of diacetone alcohol, mesityl oxide, phorone, and mesitylene; while using the mixture of the remaining C₂-C₅ alcohols for obtaining higher oxygen-containing compounds and/or non oxygen-containing hydrocarbons, including those having four and more carbon atoms in the molecule.

44. The method of claim 43, **characterized in** comprising the further steps of: extracting the mesityl oxide and phorone from the mixture of hydrocarbons obtained in the result of aldol and kroton condensation of acetone, and subsequent hydrogenation of mesityl oxide and phorone to obtain saturated isohexyl and isononyl alcohols.

45. The method of any one of claims 16, 17, 18, 20, 24, 33 and 34, **characterized in** comprising the further steps of: condensation of the unsaturated C₂ and higher hydrocarbons with C₂ and higher aldehydes into unsaturated C₄ and higher alcohols; and hydrogenation of the unsaturated C₄ and higher alcohols into the corresponding saturated C₄ and higher alcohols.

46. The method of any one of claims 18-45, **characterized in** comprising the further step of: obtaining the hydrogen used for hydrogenation from biomass, and/or by biochemical methods, and/or from the water obtained in processing of alcohols obtained by biosynthesis.

47. The method of claim 16 or 17, **characterized in** comprising the further steps of: separation of glycerin from the mixture of C3-C5 alcohols obtained in fermentation of carbohydrate substrates; condensation of glycerin either with acetaldehyde, obtained by biochemical method, to obtain glycerinacetal, or with acetone, obtained by biochemical method, to obtain glycerinketal; while the mixture of the remaining C₂-C₅ alcohols is used for obtaining higher oxygen-containing compounds and/or non oxygen-containing hydrocarbons, including those having in the molecule fore and more carbon atoms.

48. The method of any one of the previous claims, **characterized in** comprising the further steps of: preparation of synthesis gas from biomass and/or from wastes obtained in the processing of any of the products obtained in fermentation of carbohydrate substrate, into higher hydrocarbons, and/or methane produced by biochemical methods and from carbon dioxide, obtained by biochemical method; and using said synthesis gas obtained from biochemical raw material for producing non oxygen-containing hydrocarbons by Fisher-Tropsch method.

49. The method of any one of the previous claims, **characterized in** comprising the further steps of: preparation of synthesis gas from biomass and/or from wastes obtained in the processing of any of the products obtained in fermentation of carbohydrate substrate, into higher hydrocarbons, and/or methane produced by biochemical methods and from carbon dioxide, obtained by biochemical method; and using said synthesis gas obtained from biochemical raw material for producing oxygen-containing hydrocarbons by Fisher-Tropsch method.

50. The method of any one of the claims 16, 17 and 18, **characterized in** comprising the further step of: reacting unsaturated C₂ and higher hydrocarbons, obtained in the dehydration of the corresponding saturated alcohols obtained in biosynthesis, with carbon oxide obtained from biochemical raw material, and water to yield the corresponding saturated C₃ and higher alcohols.

51. The method of any one of claims 16, 17, and 18, **characterized in** comprising the further steps of: mixing of ethylene obtained in dehydration of ethanol with methanol and butylene peroxides; and treatment of the resulting mixture by telomerisation to yield a mixture of C₃-C₁₂ alcohols.

## Patentansprüche

1. Verfahren zur Herstellung von Kohlenwasserstoffen und sauerstoffhaltigen Verbindungen aus Biomasse, umfassend die Schritte: Zubereiten eines wässrigen Kohlenhydrat-Substrats mit einer Kohlenhydrat-Konzentration von 3 bis 20 %, umfassend eine Stickstoffquelle; Fermentieren des Substrats unter Verwendung von Hefe oder eines Bakteriums, ausgewählt aus Clostridium acetobutylicum und Clostridium butylicum, bis zu einer Gesamtkonzentration von 1,5 bis 10 % der folgenden Produkte C₁₋₅-Alkohole, Glycerin (Glycerol), Acetaldehyd, Essigsäure und Aceton; und Abtrennen der gewünschten Produkte von dem Fermentationsmedium, **dadurch gekennzeichnet, dass** als eine Stickstoffquelle die Aminosäuren Leucin, Isoleucin oder Valin oder eine Mischung hiervon in einer Menge zu dem wässrigen Kohlenhydrat-Substrat zugegeben wird, die eine Menge an Amino-Stickstoff von 120 bis 420 mg/l des Fermentationsmediums bereitstellt.

2. Verfahren gemäß Anspruch 1, **dadurch gekennzeichnet, dass** der Prozess der Fermentation bei einer Geschwindigkeit von 2,8 bis 4,0 l/kg je Stunde durchgeführt wird.

3. Verfahren gemäß Anspruch 1 oder 2, **dadurch gekennzeichnet, dass** das verwendete Kohlenhydrat-Substrat Rüben- oder Zuckerrohr-Molassen, saccharierte Stärke von verschiedenen Arten von Getreide oder Kartoffeln, worin die Stärke ein Säure-Stärke-Hydrolysat ist oder ein enzymatisches Stärkehydrolysat ist, ist.

4. Verfahren gemäß irgendeinem der vorstehenden Ansprüche, **dadurch gekennzeichnet, dass** der Gehalt an Amino-Stickstoff in dem Medium von 320 bis 400 mg/l beträgt.

5. Verfahren gemäß Anspruch 4, **dadurch gekennzeichnet, dass** der Gehalt von Amino-Stickstoff in dem Medium von 350 bis 370 mg/l beträgt.

6. Verfahren gemäß irgendeinem der vorstehenden Ansprüche, **dadurch gekennzeichnet, dass** es die weiteren Schritte umfasst: Kondensieren von Hefe, die in der Fermentation des Kohlenhydrat-Substrats erhalten wird, zu einem Gehalt an Trockensubstanz von 5 bis 10 %; und Autolyse des Hefe-Proteins bei 45 bis 55°C für 24 bis 48 Stunden zum Erhalt eines Autolysats, das einen Gehalt an Amino-Stickstoff von 3.000 bis 8.000 mg/l aufweist.

7. Verfahren gemäß irgendeinem der vorstehenden Ansprüche, **dadurch gekennzeichnet, dass** es die weiteren Schritte umfasst: Kondensieren von suspendierten Substanzen, die in dem Fermentationsmedium nach der Fermentation des Kohlenhydrat-Substrats enthalten sind, und Abtrennung des Alkohols hiervon, um einen Gehalt an Trockensubstanz von 5 bis 10 % zu erhalten; und entweder Säure-Hydrolyse des Proteins, das in den Substanzen enthalten ist, unter Verwendung von Schwefel- oder Salzsäuren, oder enzymatische Hydrolyse des Proteins, das in den Substanzen enthalten ist, unter Verwendung von proteolytischen enzymatischen Zubereitungen, um ein Säure-Hydrolysat von Proteinen zu erhalten, das eine Konzentration von Amino-Stickstoff von 2.000 bis 6.000 mg/l aufweist.

8. Verfahren gemäß irgendeinem der vorstehenden Ansprüche, **dadurch gekennzeichnet, dass** es die weiteren Schritte umfasst: aerobes Kultivieren von Hefe unter Verwendung der wasserlöslichen Substanzen, die in dem Fermentationsmedium nach der Fermentation des Kohlenhydrat-Substrats und der Abtrennung des Alkohols hiervon enthalten sind; Kondensieren der somit erhaltenen Hefe zu einem Gehalt an Trockensubstanz von 5 bis 10 %; und Autolyse des Hefe-Proteins bei 45 bis 55°C für 24 bis 48 Stunden, um ein Autolysat zu erhalten, das einen Gehalt an Amino-Stickstoff von 3.000 bis 8.000 mg/l aufweist.

9. Verfahren gemäß Anspruch 1 oder 2, **dadurch gekennzeichnet, dass** das Kohlenhydrat-Substrat ein Säure-Hydrolysat von Cellulose-haltigen Materialien ist.

10. Verfahren gemäß Anspruch 9, **dadurch gekennzeichnet, dass** es die weiteren Schritte umfasst: aerobes Kultivieren von Hefe mit dem Pentose-haltigen Fermentationsmedium nach der Fermentation des Kohlenhydrat-Substrats und Abtrennung des Alkohols hiervon; Kondensieren der somit erhaltenen Hefe auf einen Gehalt an Trockensubstanz von 5 bis 10 %; und Autolyse des Hefe-Proteins bei 45 bis 55°C für 24 bis 48 Stunden, um ein Autolysat von Hefeprotein zu erhalten, das einen Gehalt an Amino-Stickstoff von 3.000 bis 8.000 mg/l aufweist.

11. Verfahren gemäß irgendeinem der vorstehenden Ansprüche, **dadurch gekennzeichnet, dass** eine Mischung von Alkoholen von dem Fermentationsmedium mittels Destillation abgetrennt wird, die einen Ethanolgehalt von 96,9 bis 99,35 und einen Gehalt von C₃₋₅-Alkoholen von 0,65 bis 3,1 Vol.-% aufweist.

12. Verfahren gemäß irgendeinem der Ansprüche 1 bis 10, **dadurch gekennzeichnet, dass** eine Produktmischung von dem Fermentationsmedium abgetrennt wird, die einen Gehalt von Aceton von 25,5 bis 32,7, n-Butanol von 56,0 bis 58,5, Ethanol von 7,3 bis 8,7 %, Isopropanol von 0,4 bis 4,4, Isobutanol von 1,1 bis 1,5 und Isopentanol von 1,8 bis 2,2 Vol.-% aufweist.

13. Verfahren gemäß irgendeinem der vorstehenden Ansprüche, **dadurch gekennzeichnet, dass** es den weiteren Schritt umfasst: Verwenden von C₁₋₅-Alkoholen, Glycerin, Acetaldehyd und Aceton, die durch Biosynthese erhalten sind, in der Zubereitung eines Motor-Kraftstoffs.

14. Verfahren gemäß irgendeinem der Ansprüche 5 bis 8 und 10 bis 13, **dadurch gekennzeichnet, dass** es den weiteren Schritt umfasst: Trocknen des überschüssigen Autolysats des Hefeproteins zur Verwendung als Tierfutter.

15. Verfahren gemäß irgendeinem der Ansprüche 5 bis 8 und 10 bis 14, **dadurch gekennzeichnet, dass** es den weiteren Schritt umfasst: Biosynthese von Methan unter Verwendung von suspendierten Substanzen, erhalten in der Säure- oder enzymatischen Hydrolyse oder Autolyse von Protein mit dem überschüssigen Hydrolysat als ein Substrat.

16. Verfahren gemäß irgendeinem der vorstehenden Ansprüche, **dadurch gekennzeichnet, dass** es den weiteren Schritt umfasst: Erhalten von höheren sauerstoffhaltigen Verbindungen und/oder nicht-sauerstoffhaltigen Kohlenwasserstoffen, einschließlich derjenigen, die vier und mehr Kohlenstoffatome im Molekül aufweisen, unter Verwendung der von dem Fermentationsmedium abgetrennten Produktmischung von C₁₋₅-Alkoholen, Glycerin, Acetaldehyd und Aceton.

17. Verfahren gemäß Anspruch 16, **dadurch gekennzeichnet, dass** es den weiteren Schritt umfasst: Verwenden der in dem Verfahren gemäß Anspruch 16 erhaltenen Verbindungen in der Zubereitung von Motor-Kraftstoffen.

18. Verfahren gemäß Anspruch 16 oder 17, **dadurch gekennzeichnet, dass** es die weiteren Schritte umfasst: Dehydratieren der nach der Fermentation erhaltenen Produktmischung von C₁₋₅-Alkoholen, um ungesättigte C₂₋₅-Kohlenwasserstoffe zu erhalten; Umsetzen der ungesättigten C₂₋₅-Kohlenwasserstoffe mit Synthesegas in einer Hydroformulierungsreaktion, um Aldehyde zu erhalten; Hydrieren der Aldehyde zu einer Mischung von höheren Alkoholen, wobei alternativ die Aldehyde zunächst zu höheren ungesättigten Aldehyden kondensiert werden, die dann zu den entsprechend höheren gesättigten Alkoholen hydriert werden.

19. Verfahren gemäß Anspruch 18, **dadurch gekennzeichnet, dass** es die weiteren Schritte umfasst: Zubereiten von Synthesegas aus Biomasse und/oder aus Abfällen, die in der Verarbeitung der abgetrennten Produktmischung zu höheren Kohlenwasserstoffen erhalten werden, C₂₋₆-Säuren und/oder Methan, erhalten durch biochemische Mittel, oder Kohlendioxid, erhalten durch biochemische Mittel.

20. Verfahren gemäß irgendeinem der Ansprüche 16 bis 19, **dadurch gekennzeichnet, dass** es die weiteren Schritte umfasst: Oxidieren der nach der Fermentation abgetrennten Produktmischung von C₁₋₅-Alkoholen in der Gegenwart von Kohlendioxid, erhalten durch biochemische Mittel, zu einer Mischung von C₁₋₅-Aldehyden; Kondensieren der Aldehyde zu einer Mischung von höheren ungesättigten Aldehyden; und anschließende Hydrierung zu einer Mischung der entsprechenden höheren gesättigten Alkohole.

21. Verfahren gemäß irgendeinem der Ansprüche 16 bis 20, **dadurch gekennzeichnet, dass** es die weiteren Schritte umfasst: Dehydratieren von gesättigten C₄- und höheren Alkoholen zu den entsprechenden ungesättigten Kohlenwasserstoffen; und Hydrieren der ungesättigten Kohlenwasserstoffe zu den entsprechenden gesättigten C₄- und höheren Kohlenwasserstoffen.

22. Verfahren gemäß irgendeinem der Ansprüche 16 bis 21, **dadurch gekennzeichnet, dass** es den weiteren Schritt umfasst: Dehydratieren von gesättigten C₃- und höheren Alkoholen, um die entsprechenden Ether zu erhalten.

23. Verfahren gemäß irgendeinem der Ansprüche 16 bis 22, **dadurch gekennzeichnet, dass** es den weiteren Schritt umfasst: Umsetzen von ungesättigten C₅₋₆-Kohlenwasserstoffen von Iso-Struktur mit Methanol, um die entsprechenden Methylester zu erhalten.

24. Verfahren gemäß irgendeinem der Ansprüche 16 bis 23, **dadurch gekennzeichnet, dass** es den weiteren Schritt umfasst: Oxidieren der nach der Fermentation abgetrennten Produktmischung von C₁₋₅-Alkoholen in der Gegenwart von Kohlendioxid, erhalten durch biochemische Mittel, um eine Mischung von Aldehyden zu erhalten; Kondensieren der Mischung zu einer Mischung von höheren ungesättigten Aldehyden; Oxidieren der ungesättigten Aldehyde in der Gegenwart von Kohlendioxid, erhalten durch biochemische Mittel, zu einer Mischjung von höheren ungesättigten Säuren, und Umsetzen der Säuren mit Methanol, um die entsprechenden Methylester zu erhalten.

25. Verfahren gemäß Anspruch 23, **dadurch gekennzeichnet, dass** es die weiteren Schritte umfasst: Hydrieren der höheren ungesättigten Säuren zu höheren gesättigten Säuren; und Umsetzen der gesättigten Säuren mit Methanol, um die entsprechenden Methylester zu erhalten.

26. Verfahren gemäß irgendeinem der Ansprüche 23 bis 25, **dadurch gekennzeichnet, dass** es die weiteren Schritte umfasst: Zubereiten von Methanol unter Verwendung von Kohlendioxid, erhalten durch biochemische Mittel, Methan, erhalten durch biochemische Mittel, und Wasserstoff, erhalten aus Biomasse und/oder durch biochemische Verfahren in der Fermentation von Kohlenhydrat-Substraten, und/oder aus Wasser, das in der Verarbeitung von Alkoholen erhalten wird, die in der Biosynthese erhalten werden.

27. Verfahren gemäß Anspruch 26, **dadurch gekennzeichnet, dass** es den weiteren Schritt umfasst: Umsetzen des Methanols mit Fett-C₄- und höheren Säuren, um die entsprechenden Ester herzustellen.

28. Verfahren gemäß Anspruch 27, **dadurch gekennzeichnet, dass** es den weiteren Schritt umfasst: Oxidieren von C₄₋₅-Alkoholen aus der Produktmischung, die nach der Fermentation abgetrennt wird, um C₄- und höhere Fettsäuren zu erhalten; und/oder Biosynthese von C₄₋₆-Fettsäuren; und/oder Extraktion von Fettsäuren aus Tallöl; und/oder Verseifung von Fetten, um Fettsäuren zu erhalten.

29. Verfahren gemäß irgendeinem der Ansprüche 16 bis 19, **dadurch gekennzeichnet, dass** es die weiteren Schritte umfasst: Dehydratieren von gesättigten C₄- und höheren Alkoholen zu den entsprechenden ungesättigten C₄- und höheren Kohlenwasserstoffen; und Umsetzen der Kohlenwasserstoffe mit C₁- und höheren Fettsäuren, um die entsprechenden Ester zu erhalten.

30. Verfahren gemäß Anspruch 28, **dadurch gekennzeichnet, dass** es die weiteren Schritte umfasst: Zubereiten von C₁- und höheren Fettsäuren durch Oxidieren von C₁₋₅-Alkoholen aus der Produktmischung, die nach der Fermentation abgetrennt wird; und/oder Zubereitung von C₂₋₆-Fettsäuren mittels Biosynthese; und/oder Extraktion von Fettsäuren aus Tallöl; und/oder Zubereitung von Fettsäuren durch Verseifung von Fetten.

31. Verfahren gemäß irgendeinem der Ansprüche 18 bis 20, **dadurch gekennzeichnet, dass** es den weiteren Schritt umfasst: Umsetzen der ungesättigten C₄- und höheren Kohlenwasserstoffe, erhalten in der Dehydratierung der entsprechenden gesättigten Alkohole, mit C₂₋₅-Alkoholen, erhalten in der Biosynthese, um die entsprechenden Ether zu erhalten.

32. Verfahren gemäß Anspruch gemäß irgendeinem der Ansprüche 16 bis 20, **dadurch gekennzeichnet, dass** es die weiteren Schritte umfasst: Extraktion von Isobutan und Isopentan aus der Mischung von gesättigten Kohlenwasserstoffen; Umsetzung mit ungesättigten C₂- und höheren Kohlenwasserstoffen, erhalten in der Dehydratierung der entsprechenden gesättigten Alkohole, um gesättigte C₆- und höhere Kohlenwasserstoffe zu erhalten.

33. Verfahren gemäß Anspruch 16 oder 17, **dadurch gekennzeichnet, dass** es die weiteren Schritte umfasst: Verarbeiten von pflanzlichen und/oder tierischen Fetten und/oder Glycerin, erhalten in der Verseifung von Fetten, und/oder Glycerin, erhalten in der Biosynthese, zu n-Propanol; Mischen des n-Propanols mit C₁₋₅-Alkoholen, abgetrennt nach der Fermentation; Zubereiten von höheren, sauerstoffhaltigen Verbindungen und/oder nicht-sauerstoffhaltigen Kohlenwasserstoffen, einschließlich derjenigen mit vier und mehr Kohlenstoffen im Molekül, unter Verwendung der Mischung.

34. Verfahren gemäß Anspruch 16 oder 17, **dadurch gekennzeichnet, dass** es die weiteren Schritte umfasst: Extraktion von Glycerin, extrahiert aus einer Produktmischung von C₃₋₅-Alkoholen, erhalten in der Fermentation von Kohlenhydrat-Substraten; Dehydratierung des Glycerins zu Acrolein; Hydrieren des Acroleins zu Propionaldehyd und Propylalkohol; Kondensation des Propionaldehyds mit den C₃₋₅-Alkoholen, erhalten in der Fermentation des Kohlenhydrat-Substrats, und Propanol, erhalten in der Hydrierung von Acrolein, zu den entsprechenden Propanalen; alternativ wird Propionaldehyd zunächst zu ungesättigtem Isohexen-Aldehyd kondensiert, welches dann zu dem gesättigten Alkohol Isohexanol hydriert wird.

35. Verfahren gemäß Anspruch 16 oder 17, **dadurch gekennzeichnet, dass** es die weiteren Schritte umfasst: Extrahieren von Glycerin, extrahiert aus einer Produktmischung von C₃₋₅-Alkoholen, erhalten in der Fermentation von Kohlenhydrat-Substraten; Dehydratieren des Glycerins zu Acrolein; Kondensieren des Acroleins zu dem Acrolein-Dimer 2-Formyl-3,4-dihydro-2H-pyran; Hydrieren des Acrolein-Dimers zu Tetrahydropyran-2-methanol; während die verbleibende Mischung von C₃₋₅-Alkoholen, erhalten in der Fermentation von Kohlenhydrat-Substraten, zum Erhalt von höheren sauerstoffhaltigen Verbindungen und/oder nicht-sauerstoffhaltigen Kohlenwasserstoffen, einschließlich derjenigen, die in dem Molekül vier und mehr Kohlenstoffatome aufweisen, verwendet wird.

36. Verfahren gemäß Anspruch 16 oder 17, **dadurch gekennzeichnet, dass** es die weiteren Schritte umfasst: Extraktion von Methanol und Ethanol aus der Mischung von C₁₋₅-Alkoholen, erhalten in der Fermentation von Kohlenhydrat-Substraten; Zugeben von Methanol, hergestellt aus Kohlendioxid, erhalten in der Fermentation von Kohlenhydrat-Substraten, und Wasserstoff, erhalten aus Biomasse; Oxidation des Methanols und des Ethanols zu Formaldehyd bzw. Acetaldehyd; Kondensieren der erhaltenen Mischung von Formaldehyd und Acetaldehyd zu Acrolein; Kondensieren des Acroleins zu Acrolien-Dimer 2-Formyl-4,4-dihydro-2H-pyran; Hydrieren des Acrolein-Dimers zu Tetrahydropyran-2-methanol, während die verbleibende Mischung von C₃₋₅-Alkoholen, erhalten in der Fermentation von Kohlenhydrat-Substraten, zum Erhalt von höheren, sauerstoffhaltigen Verbindungen and/oder nicht-sauerstoffhaltigen Kohlenwasserstoffen, einschließlich derjenigen mit vier und mehr Kohlenstoffatomen im Molekül, verwendet wird.

37. Verfahren gemäß irgendeinem der Ansprüche 16, 17 und 33, **dadurch gekennzeichnet, dass** es die weiteren Schritte umfasst: Kondensieren der Mischung von C₁₋₅-Alkoholen, abgetrennt nach der Fermentation, und/oder n-Propylalkohol, erhalten aus Glycerin, um gesättigte C₆- und höhere Alkohole, gesättigte C₅- und höhere Ester, und C₂- und höhere Fettsäuren herzustellen; während jegliche verbleibende niederen Alkohole, die nicht kondensiert wurden, und gasförmige Produkte, erhalten in der Kondensation, zur Herstellung von höheren sauerstoffhaltigen Verbindungen und/oder nicht-sauerstoffhaltigen Kohlenwasserstoffen, einschließlich derjenigen, die in dem Molekül vier und mehr Kohlenstoffatome aufweisen, verwendet werden.

38. Verfahren gemäß Anspruch 37, **dadurch gekennzeichnet, dass** es ferner die Schritte umfasst: Dehydratieren der gesättigten C₆- und höheren Alkohole, erhalten in der Kondensation von C₁₋₅-Alkoholen, um ungesättigte C₆- und höhere Kohlenwasserstoffe zu erhalten; und Hydrieren der ungesättigten C₆- und höheren Kohlenwasserstoffe zu gesättigten C₆- und höheren Kohlenwasserstoffen.

39. Verfahren gemäß Anspruch 38, **dadurch gekennzeichnet, dass** es die weiteren Schritte umfasst: Umsetzen von ungesättigten C₆- und höheren Kohlenwasserstoffen, erhalten in der Dehydratierung der entsprechenden gesättigten Alkohole, mit nicht-kondensierten C₁₋₅-Alkoholen, um die entsprechenden C₇- und höheren Ether zu erhalten.

40. Verfahren gemäß Anspruch 39, **dadurch gekennzeichnet, dass** es die weiteren Schritte umfasst: Dehydratieren von nicht-kondensierten niederen C₂₋₅-Alkoholen, um ungesättigte C₂₋₅-Kohlenwasserstoffe zu erhalten; Alkylieren von Terpenen durch ungesättigte C₂₋₅-Kohlenwasserstoffe, um C₁₂- und höhere Kohlenwasserstoffe zu erhalten.

41. Verfahren gemäß Anspruch 37 oder 38, **dadurch gekennzeichnet, dass** es den weiteren Schritt umfasst: Umsetzen von C₂- und höheren Fettsäuren, erhalten in der Kondensation von C₁₋₅-Alkoholen, mit ungesättigten C₆- und höheren Kohlenwasserstoffen, erhalten in der Dehydratierung der entsprechenden gesättigten Alkohole, um die entsprechenden C₈- und höheren Ester zu erhalten.

42. Verfahren gemäß Anspruch 37, **dadurch gekennzeichnet, dass** es den weiteren Schritt umfasst: Umsetzen von C₂- und höheren Fettsäuren, erhalten in dem Prozess der Kondensation von C₁₋₅-Alkoholen, mit Terpenen, um die entsprechenden C₁₂- und höheren Ester zu erhalten.

43. Verfahren gemäß irgendeinem der vorstehenden Ansprüche, **dadurch gekennzeichnet, dass** es die weiteren Schritte umfasst: Abtrennen von Aceton aus der Mischung von C₂₋₅-Alkoholen, die in der Fermentation von Kohlenhydrat-Substraten erhalten wird; Behandlung des Acetons durch Aldol- und Kroton-Kondensation, um eine Mischung von Diacetonalkohol, Mesityloxid, Phoron und Mesitylen zu erhalten, während die Mischung der verbleibenden C₂₋₅-Alkohole zum Erhalt von höheren sauerstoffhaltigen Verbindungen und/oder nicht-sauerstoffhaltigen Kohlenwasserstoffen, einschließlich derjenigen mit vier und mehr Kohlenstoffatomen im Molekül, verwendet wird.

44. Verfahren gemäß Anspruch 43, **dadurch gekennzeichnet, dass** es die weiteren Schritte umfasst: Extrahieren des Mesityloxids und Phorons aus der Mischung von Kohlenwasserstoffen, die im Ergebnis der Aldol- und Kroton-Kondensation von Aceton erhalten wird, und anschließende Hydrierung von Mesityloxid und Phoron, um gesättigte Isohexyl-und Isononyl-Alkohole zu erhalten.

45. Verfahren gemäß irgendeinem der Ansprüche 16, 17, 18, 20, 24, 33 und 34, **dadurch gekennzeichnet, dass** es die weiteren Schritte umfasst: Kondensieren der ungesättigten C₂- und höheren Kohlenwasserstoffe mit C₂- und höheren Aldehyden zu ungesättigten C₄- und höheren Alkoholen; und Hydrieren der ungesättigten C₄- und höheren Alkohole zu den entsprechenden gesättigten C₄- und höheren Alkoholen.

46. Verfahren gemäß irgendeinem der Ansprüche 18 bis 45, **dadurch gekennzeichnet, dass** es den weiteren Schritt umfasst: Erhalten des Wasserstoffs, das für die Hydrierung verwendet wird, aus Biomasse und/oder durch biochemische Verfahren und/oder aus dem Wasser, das in der Verarbeitung von Alkoholen, die durch Biosynthese erhalten sind, erhalten wird.

47. Verfahren gemäß Anspruch 16 oder 17, **dadurch gekennzeichnet, dass** es die weiteren Schritte umfasst: Abtrennen von Glycerin aus der Mischung C₃₋₅-Alkoholen, die in der Fermentation von Kohlenhydrat-Substraten erhalten wird; Kondensation von Glycerin, entweder mit Acetaldehyd, erhalten durch ein biochemisches Verfahren, um Glycerinacetal zu erhalten, oder mit Aceton, erhalten durch ein biochemisches Verfahren, um Glycerinketal zu erhalten; während die Mischung der verbleibenden C₂₋₅-Alkohole zum Erhalt von höheren sauerstoffhaltigen Verbindungen und/oder nicht-sauerstoffhaltigen Kohlenwasserstoffen, einschließlich derjenigen mit vier und mehr Kohlenstoffatomen im Molekül, verwendet wird.

48. Verfahren gemäß irgendeinem der vorstehenden Ansprüche, **dadurch gekennzeichnet, dass** es die weiteren Schritte umfasst: Zubereiten von Synthesegas aus Biomasse und/oder aus Abfällen, die in der Verarbeitung von irgendeinem der Produkte, die in der Fermentation von Kohlenhydrat-Substrat erhalten werden, zu höheren Kohlenwasserstoffen erhalten werden, und/oder Methan, hergestellt durch biochemische Verfahren, und aus Kohlendioxid, erhalten durch biochemisches Verfahren; und Verwenden des Synthesegases, das aus biochemischem Ausgangsmaterial erhalten ist, zur Herstellung von nicht-sauerstoffhaltigen Kohlenwasserstoffen durch ein Fisher-Tropsch-Verfahren.

49. Verfahren gemäß irgendeinem der vorstehenden Ansprüche, **dadurch gekennzeichnet, dass** es die weiteren Schritte umfasst: Zubereiten von Synthesegas aus Biomasse und/oder aus Abfällen, die in der Verarbeitung von irgendeinem der Produkte, die in der Fermentation von Kohlenhydrat-Substrat erhalten werden, zu höheren Kohlenwasserstoffen erhalten werden, und/oder Methan, hergestellt durch biochemische Verfahren, und aus Kohlendioxid, erhalten durch biochemisches Verfahren; und Verwenden des Synthesegases, das aus biochemischem Ausgangsmaterial erhalten ist, zur Herstellung von sauerstoffhaltigen Kohlenwasserstoffen durch ein Fisher-Tropsch-Verfahren.

50. Verfahren gemäß irgendeinem der Ansprüche 16, 17 und 18, **dadurch gekennzeichnet, dass** es den weiteren Schritt umfasst: Umsetzen von ungesättigten C₂- oder höheren Kohlenwasserstoffen, erhalten in der Dehydratierung der entsprechenden gesättigten Alkohole, die in der Biosynthese erhalten wurden, mit Kohlendioxid, erhalten aus biochemischem Ausgangsmaterial, und Wasser, um die entsprechenden gesättigten C₃- und höheren Alkohole zu erhalten.

51. Verfahren gemäß irgendeinem der Ansprüche 16, 17 und 18, **dadurch gekennzeichnet, dass** es die weiteren Schritte umfasst: Mischen von Ethylen, erhalten in der Dehydratierung von Ethanol, mit Methanol und Butylen-Peroxiden; und Behandeln der resultierenden Mischung durch Telomerisation, um eine Mischung von C₃₋₁₂-Alkoholen zu erhalten.

## Revendications

1. Procédé de production d'hydrocarbures et de composés contenant de l'oxygène à partir de biomasse, comprenant les étapes de : préparation d'un substrat glucidique aqueux avec une concentration en glucides de 3-20 % comprenant une source d'azote ; fermentation du substrat avec une levure ou une bactérie choisie parmi Clostridium acetobutylicum et Clostridium butylicum jusqu'à une concentration globale de 1,5 - 10 % des produits suivants C₁-C₅ alcools, glycérine (glycérol), acétaldéhyde, acide acétique et acétone; et séparation des produits souhaités du milieu de fermentation, **caractérisé en ce que**, comme source d'azote, les acides aminés leucine, isoleucine ou valine, ou un mélange de ceux-ci est ajouté au substrat glucidique aqueux en une quantité procurant une teneur d'azote d'amino de 120 à 420 mg/l de milieu de fermentation.

2. Procédé selon la revendication 1, **caractérisé en ce que** le processus de fermentation est réalisé à une vitesse de 2,8-4,0 l/kg par heure.

3. Procédé selon la revendication 1 ou 2, **caractérisé en ce que** le substrat glucidique utilisé est de la mélasse de betterave ou de canne à sucre, de l'amidon saccharifié de différents types de grains ou de pommes de terre, où l'amidon est un hydrolysat d'amidon acide ou un hydrolysat d'amidon enzymatique.

4. Procédé selon l'une quelconque des revendications précédentes, **caractérisé en ce que** la teneur d'azote d'amino dans le milieu est de 320 à 400 mg/l.

5. Procédé selon la revendication 4, **caractérisé en ce que** la teneur d'azote d'amino dans le milieu est de 350 à 370 mg/l.

6. Procédé selon l'une quelconque des revendications précédentes, **caractérisé en ce qu'**il comprend les étapes supplémentaires de: condensation de la levure obtenue dans la fermentation de substrat glucidique jusqu'à une teneur en substance sèche de 5-10%; et autolyse de la protéine de levure à 45-55°C pendant 24-48 heures pour obtenir un autolysat présentant une teneur d'azote d'amino de 3000-8000 mg/l.

7. Procédé selon l'une quelconque des revendications précédentes, **caractérisé en ce qu'**il comprend les étapes supplémentaires de: condensation de substances en suspension contenues dans le milieu de fermentation après la fermentation de substrat glucidique et séparation d'alcool de celui-ci et jusqu'à une teneur en substance sèche de 5-10%; et, hydrolyse acide de la protéine contenue dans lesdites substances avec des acides sulfurique ou chlorhydrique ou hydrolyse enzymatique de la protéine contenue dans lesdites substances au moyen de préparations enzymatiques protéolytiques, pour obtenir un hydrolysat acide de protéines présentant une teneur d'azote d'amino de 2000-6000 mg/l.

8. Procédé selon l'une quelconque des revendications précédentes, **caractérisé en ce qu'**il comprend les étapes supplémentaires de: culture aérobie de levure en utilisant les substances solubles dans l'eau contenues dans le milieu de fermentation après la fermentation de substrat glucidique et séparation d'alcool de celui-ci; condensation de la levure ainsi obtenue jusqu'à une teneur en substance sèche de 5-10%; et autolyse de la protéine de levure à 45-55°C pendant 24-48 heures pour obtenir un autolysat présentant une teneur d'azote d'amino de 3000-8000 mg/l.

9. Procédé selon la revendication 1 ou 2, **caractérisé en ce que** le substrat glucidique est un hydrolysat acide de matières contenant de la cellulose.

10. Procédé selon la revendication 9, **caractérisé en ce qu'**il comprend les étapes supplémentaires de: culture aérobie de levure avec le milieu de fermentation contenant du pentose après la fermentation de substrat glucidique et séparation d'alcool de celui-ci; condensation de la levure ainsi obtenue jusqu'à une teneur en substance sèche de 5-10%; et autolyse de la protéine de levure à 45-55°C pendant 24-48 heures pour obtenir un autolysat de protéine de levure présentant une teneur d'azote d'amino de 3000-8000 mg/l.

11. Procédé selon l'une quelconque des revendications précédentes, **caractérisé en ce qu'**un mélange d'alcools est séparé du milieu de fermentation par distillation, présentant une teneur en éthanol de 96,9-99,35 une teneur en C₃-C₅ alcools de 0,65-3,1% en volume.

12. Procédé selon l'une quelconque des revendications 1-10, **caractérisé en ce qu'**un mélange de produits est séparé du milieu de fermentation, présentant une teneur d'acétone de 25,5-32,7, de n-butanol de 56,0-58,5, d'éthanol de 7,3-8,7%, d'isopropanol de 0,4-4,4, d'isobutanol de 1,1-1,5 et d'isopentanol de 1,8-2,2% en volume.

13. Procédé selon l'une quelconque des revendications précédentes **caractérisé en ce qu'**il comprend l'étape supplémentaire de: utilisation de C₁-C₅ alcools, glycérine, acétaldéhyde, et acétone obtenus dans la biosynthèse dans la préparation d'un carburant pour moteur.

14. Procédé selon l'une quelconque des revendications 5-8 et 10-13, **caractérisé en ce qu'**il comprend l'étape supplémentaire de: séchage de l'autolysat en excès de la protéine de levure pour une utilisation comme aliment pour animaux.

15. Procédé selon l'une quelconque des revendications 5-8 et 10-14, **caractérisé en ce qu'**il comprend l'étape supplémentaire de: biosynthèse de méthane en utilisant les substances en suspension obtenues dans l'hydrolyse acide ou enzymatique ou l'autolyse de protéine avec l'hydrolysat en excès comme substrat.

16. Procédé selon l'une quelconque des revendications précédentes, **caractérisé en ce qu'**il comprend l'étape supplémentaire de: obtention de composés contenant de l'oxygène supérieurs et/ou d'hydrocarbures ne contenant pas d'oxygène supérieurs, incluant ceux ayant quatre atomes de carbone et plus dans la molécule en utilisant le mélange de produits de C₁-C₅ alcools, glycérine, acétaldéhyde et acétone séparé du milieu de fermentation.

17. Procédé selon la revendication 16, **caractérisé en ce qu'**il comprend l'étape supplémentaire de: utilisation des composés obtenus dans le procédé selon la revendication 16 dans la préparation de carburants pour moteur.

18. Procédé selon la revendication 16 ou 17, **caractérisé en ce qu'**il comprend les étapes supplémentaires de: déshydratation du mélange de produits de C₁-C₅ alcools séparé après la fermentation pour obtenir des C₂-C₅ hydrocarbures insaturés; réaction desdits C₂-C₅ hydrocarbures insaturés avec du gaz de synthèse dans une réaction d'hydroformylation pour obtenir des aldéhydes ; hydrogénation desdits aldéhydes en un mélange d'alcools supérieurs, à titre d'alternative lesdits aldéhydes sont d'abord condensés en aldéhydes insaturés supérieurs qui sont ensuite hydrogénés en les alcools saturés supérieurs correspondants.

19. Procédé selon la revendication 18 **caractérisé en ce qu'**il comprend les étapes supplémentaires de: préparation de gaz de synthèse à partir de biomasse et/ou à partir de déchets obtenus dans la transformation du mélange de produits séparé en hydrocarbures supérieurs, C₂-C₆ acides et/ou méthane obtenu par des moyens biochimiques ou dioxyde de carbone obtenu par des moyens biochimiques.

20. Procédé selon l'une quelconque des revendications 16-19, **caractérisé en ce qu'**il comprend les étapes supplémentaires de: oxydation du mélange de produits de C₁-C₅ alcools séparé après la fermentation en présence de dioxyde de carbone, obtenu par des moyens biochimiques, en un mélange de C₁-C₅ aldéhydes; condensation desdits aldéhydes en un mélange d'aldéhydes insaturés supérieurs; et hydrogénation subséquente en un mélange des alcools saturés supérieurs correspondants.

21. Procédé selon l'une quelconque des revendications 16-20, **caractérisé en ce qu'**il comprend les étapes supplémentaires de: déshydratation d'alcools saturés en C₄ et supérieurs en les hydrocarbures insaturés correspondants ; et hydrogénation desdits hydrocarbures insaturés en les hydrocarbures saturés en C₄ et supérieurs correspondants.

22. Procédé selon l'une quelconque des revendications 16-21, **caractérisé en ce qu'**il comprend l'étape supplémentaire de: déshydratation d'alcools saturés en C₃ et supérieurs pour obtenir les éthers correspondants.

23. Procédé selon l'une quelconque des revendications 16-22, **caractérisé en ce qu'**il comprend l'étape supplémentaire de: réaction de C₅-C₆ hydrocarbures insaturés de structure iso avec le méthanol pour obtenir les méthyléthers correspondants.

24. Procédé selon l'une quelconque des revendications 16-23, **caractérisé en ce qu'**il comprend l'étape supplémentaire de: oxydation du mélange de produits de C₁-C₅ alcools séparé après la fermentation en présence de dioxyde de carbone, obtenu par des moyens biochimiques, pour obtenir un mélange d'aldéhydes; condensation dudit mélange en un mélange d'aldéhydes insaturés supérieurs; oxydation desdits aldéhydes insaturés en présence de dioxyde de carbone, obtenu par des moyens biochimiques, en un mélange d'acides insaturés supérieurs; et réaction desdits acides avec le méthanol pour obtenir les méthylesters correspondants.

25. Procédé selon la revendication 23, **caractérisé en ce qu'**il comprend les étapes supplémentaires de: hydrogénation des acides insaturés supérieurs en acides saturés supérieurs; et réaction desdits acides saturés avec le méthanol pour obtenir les méthylesters correspondants.

26. Procédé selon l'une quelconque des revendications 23-25, **caractérisé en ce qu'**il comprend les étapes supplémentaires de: préparation de méthanol avec du dioxyde de carbone obtenu par des moyens biochimiques, du méthane obtenu par des moyens biochimiques, et de l'hydrogène obtenu à partir de biomasse et/ou par des procédés biochimiques dans la fermentation de substrats glucidiques, et/ou à partir de l'eau obtenue dans la transformation des alcools obtenus dans la biosynthèse.

27. Procédé selon la revendication 26, **caractérisé en ce qu'**il comprend l'étape supplémentaire de: réaction du méthanol avec des acides gras en C₄ et supérieurs pour produire les esters correspondants.

28. Procédé selon la revendication 27, **caractérisé en ce qu'**il comprend l'étape supplémentaire de: oxydation de C₄-C₅ alcools provenant du mélange de produits séparé après la fermentation pour obtenir des acides gras en C₄ et supérieurs; et/ou biosynthèse de C₄-C₆ acides gras; et/ou extraction d'acides gras du tallol; et/ou saponification de graisses pour obtenir des acides gras.

29. Procédé selon l'une quelconque des revendications 16-19, **caractérisé en ce qu'**il comprend les étapes supplémentaires de: déshydratation d'alcools saturés en C₄ et supérieurs en les hydrocarbures insaturés en C₄ et supérieurs correspondants; et réaction desdits hydrocarbures avec des acides gras en C₁ et supérieurs pour obtenir les esters correspondants.

30. Procédé selon la revendication 28, **caractérisé en ce qu'**il comprend les étapes supplémentaires de: préparation d'acides gras en C₁ et supérieurs par oxydation de C₁-C₅ alcools provenant du mélange de produits séparé après la fermentation; et/ou préparation de C₂-C₆ acides gras via biosynthèse; et/ou extraction d'acides gras du tallol; et/ou préparation d'acides gras par saponification de graisses.

31. Procédé selon l'une quelconque des revendications 18-20, **caractérisé en ce qu'**il comprend l'étape supplémentaire de: réaction des hydrocarbures insaturés en C₄ et supérieurs, obtenus dans la déshydratation des alcools saturés correspondants, avec des C₂-C₅ alcools obtenus dans la biosynthèse pour obtenir les éthers correspondants.

32. Procédé selon l'une quelconque des revendications 16-20, **caractérisé en ce qu'**il comprend les étapes supplémentaires de: extraction d'isobutane et d'isopentane à partir du mélange d'hydrocarbures saturés; réaction avec des hydrocarbures insaturés en C₂ et supérieurs obtenus dans la déshydratation des alcools saturés correspondants pour obtenir des hydrocarbures saturés en C₆ et supérieurs.

33. Procédé selon la revendication 16 ou 17, **caractérisé en ce qu'**il comprend les étapes supplémentaires de: transformation de graisses végétales et/ou animales, et/ou de la glycérine obtenue dans la saponification de graisses, et/ou de la glycérine obtenue dans la biosynthèse en alcool n-propylique; mélange dudit alcool n-propylique avec des C₁-C₅ alcools séparés après la fermentation; préparation de composés contenant de l'oxygène supérieurs et/ou d'hydrocarbures ne contenant pas d'oxygène supérieurs, y compris ceux ayant quatre atomes de carbone et plus dans la molécule au moyen dudit mélange.

34. Procédé selon la revendication 16 ou 17, **caractérisé en ce qu'**il comprend les étapes supplémentaires de: extraction de la glycérine extraite d'un mélange de produits de C₃-C₅ alcools obtenu dans la fermentation de substrats glucidiques; déshydratation de ladite glycérine en acroléine; hydrogénation de l'acroléine en aldéhyde propionique et alcool propylique; condensation dudit aldéhyde propionique avec les C₃-C₅ alcools obtenus dans la fermentation de substrats glucidiques, et le propanol obtenu dans l'hydrogénation de l'acroléine, en les propanals correspondants; à titre d'alternative, l'aldéhyde propionique est d'abord condensé en isohexène aldéhyde insaturé, qui est ensuite hydrogéné en l'alcool saturé isohexanol.

35. Procédé selon la revendication 16 ou 17, **caractérisé en ce qu'**il comprend les étapes supplémentaires de: extraction de la glycérine extraite d'un mélange de produits de C₃-C₅ alcools obtenu dans la fermentation de substrats glucidiques; déshydratation de ladite glycérine en acroléine; condensation de l'acroléine en le dimère d'acroléine 2-formyl-3,4-dihydro-2H-pyrane; hydrogénation du dimère d'acroléine en tétrahydropyrane-2-méthanol; tout en utilisant le mélange restant de C₃-C₅ alcools obtenu dans la fermentation de substrats glucidiques pour obtenir des composés contenant de l'oxygène supérieurs et/ou des hydrocarbures ne contenant pas d'oxygène supérieurs, y compris ceux ayant dans la molécule quatre atomes de carbone et plus.

36. Procédé selon la revendication 16 ou 17, **caractérisé en ce qu'**il comprend les étapes supplémentaires de: extraction de méthanol et d'éthanol à partir du mélange de C₁-C₅ alcools obtenu dans la fermentation de substrats glucidiques; addition de méthanol, produit à partir de dioxyde de carbone obtenu dans la fermentation de substrats glucidiques, et d'hydrogène, dérivé de biomasse; oxydation dudit méthanol et éthanol en formaldéhyde et acétaldéhyde, respectivement; condensation du mélange de formaldéhyde et d'acétaldéhyde obtenu en acroléine; condensation de l'acroléine en le dimère d'acroléine 2-formyl-4,4-dihydro-2H-pyrane; hydrogénation du dimère d'acroléine en tétrahydro-pyrane-2-méthanol, tandis que le mélange restant de C₃-C₅ alcools obtenu dans la fermentation de substrats glucidiques est utilisé pour obtenir des composés contenant de l'oxygène supérieurs et/ou des hydrocarbures ne contenant pas d'oxygène supérieurs, y compris ceux ayant quatre atomes de carbone et plus dans la molécule.

37. Procédé selon l'une quelconque des revendications 16, 17 et 33, **caractérisé en ce qu'**il comprend les étapes supplémentaires de: condensation du mélange de C₁-C₅ alcools séparé après la fermentation, et/ou d'alcool n-propylique, obtenu à partir de la glycérine, pour produire des alcools saturés en C₆ et supérieurs, des esters saturés en C₅ et supérieurs et des acides gras en C₂ et supérieurs; tout en utilisant tout alcool inférieur restant, qui ne s'est pas condensé, et les produits gazeux, obtenus dans la condensation, pour produire des composés contenant de l'oxygène supérieurs et/ou des hydrocarbures ne contenant pas d'oxygène supérieurs, y compris ceux ayant dans la molécule quatre atomes de carbone et plus.

38. Procédé selon la revendication 37, **caractérisé en ce qu'**il comprend les étapes supplémentaires de: déshydratation des alcools saturés en C₆ et supérieurs, obtenu dans la condensation de C₁-C₅ alcools, pour obtenir des hydrocarbures insaturés en C₆ et supérieurs; et hydrogénation desdits hydrocarbures insaturés en C₆ et supérieurs en hydrocarbures saturés en C₆ et supérieurs.

39. Procédé selon la revendication 38, **caractérisé en ce qu'**il comprend les étapes supplémentaires de: réaction d'hydrocarbures insaturés en C₆ et supérieurs, obtenus dans la déshydratation des alcools saturés correspondants, avec des C₁-C₅ alcools non condensés pour obtenir les éthers en C₇ et supérieurs correspondants.

40. Procédé selon la revendication 39, **caractérisé en ce qu'**il comprend les étapes supplémentaires de: déshydratation d'alcools inférieurs en C₂-C₅ non condensés pour obtenir des C₂-C₅ hydrocarbures insaturés; alkylation de terpènes par des C₂-C₅ hydrocarbures insaturés pour obtenir des hydrocarbures en C₁₂ et supérieurs.

41. Procédé selon la revendication 37 ou 38, **caractérisé en ce qu'**il comprend l'étape supplémentaire de: réaction des acides gras en C₂ et supérieurs, obtenus dans la condensation de C₁-C₅ alcools, avec des hydrocarbures insaturés en C₆ et supérieurs, obtenus dans la déshydratation des alcools saturés correspondants, pour obtenir les esters en C₈ et supérieurs correspondants.

42. Procédé selon la revendication 37, **caractérisé en ce qu'**il comprend l'étape supplémentaire de: réaction des acides gras en C₂ et supérieurs, obtenus dans le traitement de condensation de C₁-C₅ alcools, avec des terpènes pour obtenir les esters en C₁₂ et supérieurs correspondants.

43. Procédé selon l'une quelconque des revendications précédentes, **caractérisé en ce qu'**il comprend les étapes supplémentaires de: séparation de l'acétone du mélange de C₂-C₅ alcools obtenu dans la fermentation de substrats glucidiques; traitement de l'acétone par aldolisation et crotonisation pour obtenir un mélange de diacétone alcool, oxyde de mésityle, phorone et mésitylène; tout en utilisant le mélange des C₂-C₅ alcools restants pour obtenir des composés contenant de l'oxygène supérieurs et/ou des hydrocarbures ne contenant pas d'oxygène supérieurs, y compris ceux ayant quatre atomes de carbone et plus dans la molécule.

44. Procédé selon la revendication 43, **caractérisé en ce qu'**il comprend les étapes supplémentaires de: extraction de l'oxyde de mésityle et de la phorone du mélange d'hydrocarbures obtenu dans le résultat de l'aldolisation et de la crotonisation de l'acétone, et hydrogénation subséquente de l'oxyde de mésityle et de la phorone pour obtenir les alcools saturés isohexylique et isononylique.

45. Procédé selon l'une quelconque des revendications 16, 17, 18, 20, 24, 33 et 34, **caractérisé en ce qu'**il comprend les étapes supplémentaires de: condensation des hydrocarbures insaturés en C₂ et supérieurs avec des aldéhydes en C₂ et supérieurs en alcools insaturés en C₄ et supérieurs; et hydrogénation des alcools insaturés en C₄ et supérieurs en les alcools saturés en C₄ et supérieurs correspondants.

46. Procédé selon l'une quelconque des revendications 18-45, **caractérisé en ce qu'**il comprend l'étape supplémentaire de: obtention de l'hydrogène utilisé pour l'hydrogénation à partir de biomasse, et/ou par des procédés biochimiques, et/ou à partir de l'eau obtenue dans la transformation d'alcools obtenus par biosynthèse.

47. Procédé selon la revendication 16 ou 17, **caractérisé en ce qu'**il comprend les étapes supplémentaires de: séparation de glycérine du mélange de C3-C5 alcools obtenu dans la fermentation de substrats glucidiques; condensation de la glycérine avec l'acétaldéhyde, obtenu par un procédé biochimique, pour obtenir du glycérine-acétal, ou avec l'acétone, obtenue par un procédé biochimique, pour obtenir du glycérine-cétal; tandis que le mélange des C₂-C₅ alcools restants est utilisé pour obtenir des composés contenant de l'oxygène supérieurs et/ou des hydrocarbures ne contenant pas d'oxygène supérieurs, y compris ceux ayant dans la molécule quatre atomes de carbone et plus.

48. Procédé selon l'une quelconque des revendications précédentes, **caractérisé en ce qu'**il comprend les étapes supplémentaires de: préparation de gaz de synthèse à partir de biomasse et/ou à partir de déchets obtenus dans la transformation de l'un quelconque des produits obtenus dans la fermentation de substrat glucidique, en hydrocarbures supérieurs, et/ou de méthane produit par des procédés biochimiques et à partir de dioxyde de carbone, obtenu par un procédé biochimique; et l'utilisation dudit gaz de synthèse obtenu à partir de matière première biochimique pour produire des hydrocarbures ne contenant pas d'oxygène supérieurs par le procédé de Fisher-Tropsch.

49. Procédé selon l'une quelconque des revendications précédentes, **caractérisé en ce qu'**il comprend les étapes supplémentaires de: préparation de gaz de synthèse à partir de biomasse et/ou à partir de déchets obtenus dans la transformation de l'un quelconque des produits obtenus dans la fermentation de substrat glucidique, en hydrocarbures supérieurs, et/ou de méthane produit par des procédés biochimiques et à partir de dioxyde de carbone, obtenu par un procédé biochimique; et utilisation dudit gaz de synthèse obtenu à partir de matière première biochimique pour produire des hydrocarbures contenant de l'oxygène par le procédé de Fisher-Tropsch.

50. Procédé selon l'une quelconque des revendications 16, 17 et 18, **caractérisé en ce qu'**il comprend l'étape supplémentaire de: réaction d'hydrocarbures insaturés en C₂ et supérieurs, obtenus dans la déshydratation des alcools saturés correspondants obtenus dans la biosynthèse, avec de l'oxyde de carbone obtenu à partir de matière première biochimique, et d'eau pour produire les alcools saturés en C₃ et supérieurs correspondants.

51. Procédé selon l'une quelconque des revendications 16, 17 et 18, **caractérisé en ce qu'**il comprend les étapes supplémentaires de: mélange de l'éthylène obtenu dans la déshydratation de l'éthanol avec du méthanol et des peroxydes de butylène; et traitement du mélange résultant par télomérisation pour produire un mélange de C₃-C₁₂ alcools.
